# EUROPEAN PATENT SPECIFICATION

(11) **EP 2 271 665 B1**
(45) Date of publication and mention of the grant of the patent: **18.01.2012**
(21) Application number: 09728150.5
(22) Date of filing: 18.03.2009
(51) Int. Cl.: C07K 14/36, C07K 7/08, A61K 38/10, A61P 31/04, A61P 31/12, C12P 21/02

(54) **HIGHLY BRIDGED PEPTIDES FROM ACTINOMADURA NAMIBIENSIS**
STARK VERBRÜCKTE PEPTIDE AUS DER ACTINOMADURA NAMIBIENSIS
PEPTIDES HAUTEMENT PONTÉS PROVENANT D'ACTINOMADURA NAMIBIENSIS

(30) Priority: 02.04.2008 EP 08290324
(43) Date of publication of application: 12.01.2011
(73) Proprietor: SANOFI, 75013 Paris (FR)
(72) Inventor: BROENSTRUP, Mark, 65926 Frankfurt am Main (DE); GUEHRING, Hans, 65926 Frankfurt am Main (DE); HOFFMANN, Holger, 65926 Frankfurt an Main (DE); WINK, Joachim, 65926 Franckfurt am Main (DE); SUESSMUTH, Roderich, 10629 Berlin-Charlottenburg (DE); SCHMIEDERER, Timo, 10587 Berlin (DE)
(74) Representative: Kujath, Eckard
(86) International application number: PCT/EP2009/001982
(87) International publication number: WO 2009/121483

(56) References cited:
- WO-A-2008/040469
- MARAZZI ALESSANDRA ET AL: "Antibiotics GE23077, novel inhibitors of bacterial RNA polymerase II. Structure elucidation" JOURNAL OF ANTIBIOTICS, JAPAN ANTIBIOTICS RESEARCH ASSOCIATION, TOKYO, JP, vol. 58, no. 4, 1 April 2005 (2005-04-01), pages 260-267, XP002434109 ISSN: 0021-8820
- GOLDSTEIN B P ET AL: "A-40926 A NEW GLYCOPEPTIDE ANTIBIOTIC WITH ANTI-NEISSERIA ACTIVITY" ANTIMICROBIAL AGENTS AND CHEMOTHERAPY, AMERICAN SOCIETY FOR MICROBIOLOGY, WASHINGTON, DC, US, vol. 31, no. 12, 1 January 1987 (1987-01-01), pages 1961-1966, XP002434110 ISSN: 0066-4804
- SÜSSMUTH R: "Charakterisierung und molekularbiologische Handhabung eines bakteriellen Actinomadura-Stamms zur Aufklärung der Labyrinthopeptin- Biosynthese" INTERNET CITATION, [Online] XP002466709 Retrieved from the Internet: URL:http://www.alstep.tu-berlin.de/wissdoc s/suessmuth_actinom.pdf> [retrieved on 2007-05-18]

## Description

Several highly bridged peptides are known in the literature, for example conopeptides isolated from cone snails (for a review see e.g. Terlau & Olivera, Physiol. Rev. 2004, 84, 41-68) or the so-called lantibiotics (Chatterjee et al., Chem. Rev. 2005, 105, 633-683) from Gram-positive bacteria source. The said peptides have various utilities. The lantibiotic nisin, for example, is used as a food preservative since many years.

The conopeptides are useful for the treatment of pain, diabetes, multiple sclerosis and cardiovascular diseases and currently undergo preclinical or clinical development. Examples of conopeptides are α-GI (sequence: ECCNPACGRHYSC*, *amidated, connectivity: 1-3,2-4) and α-GID (sequence: IRγCCSNPACRVNNOHVC, connectivity: 1-3,2-4), wherein O/Hyp is hydroxyproline and the connectivity indicates the position of the cysteine involved in each specific disulphide bonds, for example, first to third and second to fourth as in α-GID:

A novel group of highly bridged peptides named Labyrinthopeptins has been discovered recently (European patent application EP06020980.6). The so-called Labyrinthopeptins exhibit a unique bridging motif across their peptide chain, as illustrated by the compound in formula below:

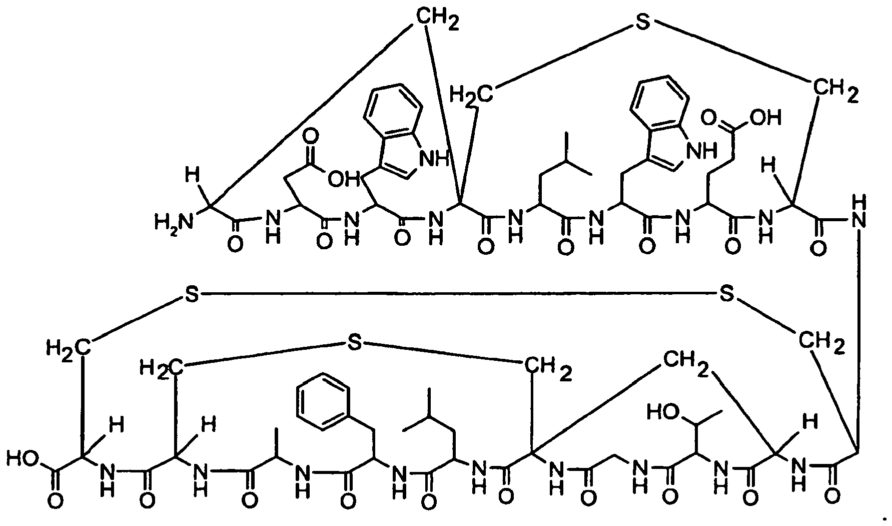

It has now been found that further highly bridged peptides of the Labyrinthopeptin class can be isolated from microorganism strain *Actinomadura namibiensis* (DSM 6313). The compounds are distinctly different from the Labyrinthopeptin derivatives as described in patent application EP06020980.6.

Some strains of *Actinomadura* are known to produce peptides, useful as antibacterial agents.

For example, Marazzi et al (Journal of Antibiotics, Vol. 58, no. 4, 260-267) describes a bacterial agent consisting in a mixture four major factors. The structural determination of this complex reveals that all factors are cyclic heptapeptides containing three natural and four unusual amino acids. Goldstein et al (Antimicrobial Agents and chemotherapy, vol. 31, no. 12, 1961-1966) described a complex of four main factors produced by an actinomycete of the genus *Actinomadura,* containing a fatty acid as part of a glycolipid attached to the peptide backbone. The activity of this peptide is similar to vancomysin or teicoplanin.

The object of the present invention is a compound produced by an *Actinomadura,* having a specific formula.

An embodiment of the present invention is a compound of the formula (I)

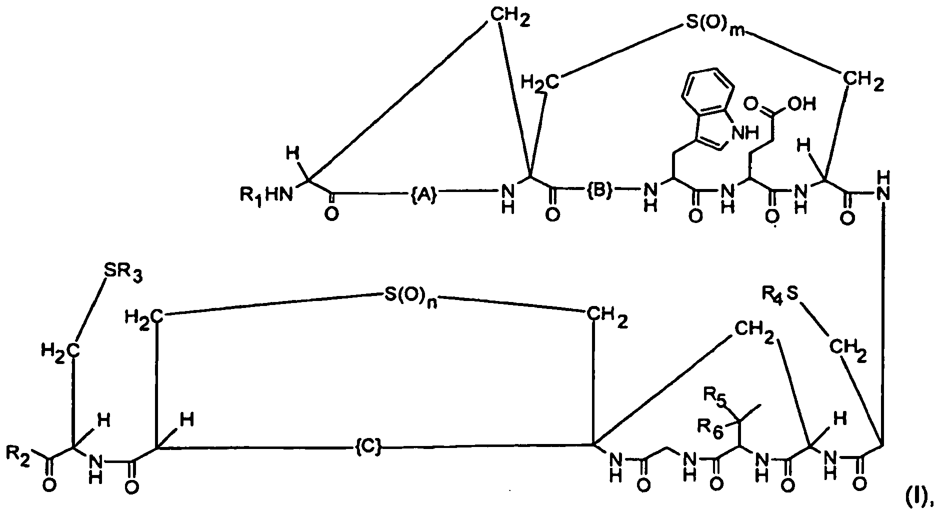

wherein
{A} is a group selected from 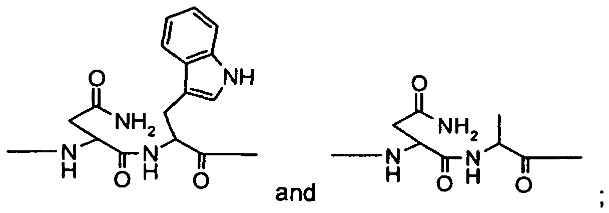
{B} is a group selected from 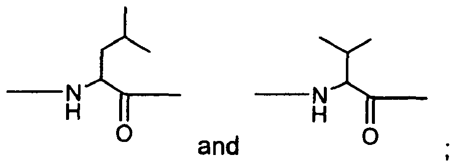
{C} is a group selected from 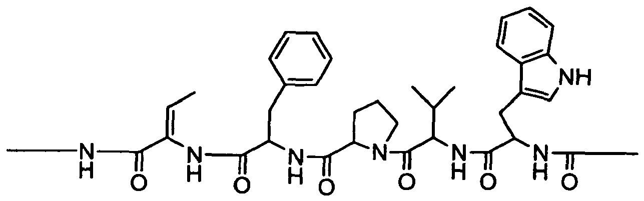 and 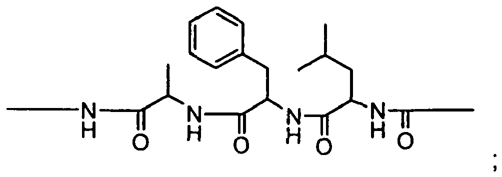 R₁ is a group R₁' or a group 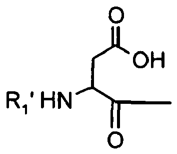
wherein R₁' is H, C(O)-(C₁-C₆)alkyl or C(O)-O-(C₁-C₆)alkyl;
R₂ is OH, NH₂, NH-(C₁-C₆)alkyl, NH-(C₁-C₄)alkylene-phenyl or NH-(C₁-C₄)alkylene-pyridyl;
R₃ and R₄ are independently of each other H, (C₁-C₆)alkyl, (C₁-C₆)alkylene-C(O)NH₂, (C₁-C₆)alkylene-C(O)NH(C₁-C₄)alkyl or (C₁-C₆)alkylene-C(O)N[(C₁-C₄)alkyl]₂, or R₃ and R₄ together with the S atoms to which they are attached form a disulfide group S-S;
R₅ and R₆ are independently of each other H or OH, or R₅ and R₆ together are =O;
m and n are independently of one another 0, 1 or 2;
with the proviso that if
{A} is 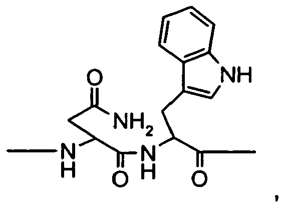
{B} is 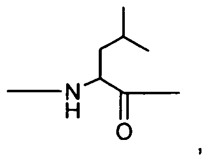 and
{C} is 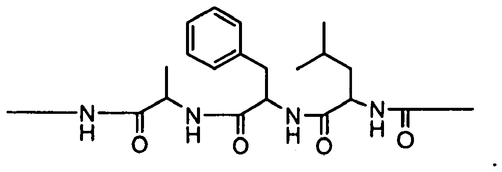
R₃ and R₄ may not form a disulfide group S-S together with the S atoms to which they are attached;
in any stereochemical form, or a mixture of any stereochemical forms in any ratio, or a physiologically tolerable salt thereof.

Preferably,
{A} is 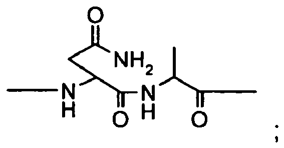
{B} is 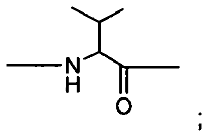
{C} is

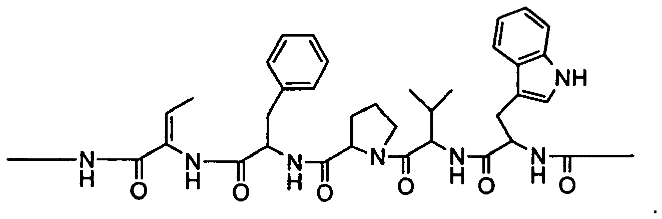

Further preferred,
{A} is 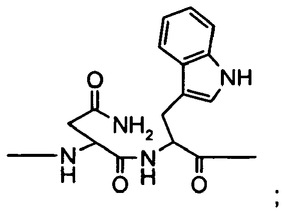
{B} is 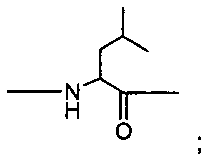 and
{C} is 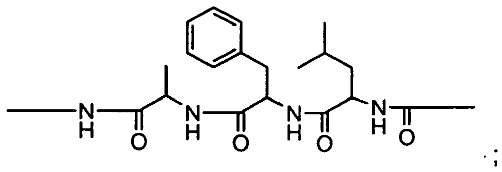 and
R₁ is preferably a group R₁'.
R₁' is preferably H.
R₂ is preferably OH.
R₃ and R₄ are preferably independently of each other H, (C₁-C₆)alkyl, (C₁-C₆)alkylene-C(O)NH₂, or form a disulfide group S-S together with the S atoms to which they are attached. More preferred, R₃ and R₄ are H or form a disulfide group S-S together with the S atoms to which they are attached. Most preferred, R₃ and R₄ form a disulfide group S-S together with the S atoms to which they are attached.
R₅ and R₆ are preferably H or OH wherein if R₅ is OH then R₆ is H, and if R₅ is H then R₆ is OH, or R₅ and R₆ together are =O. More preferred, R₅ is OH and R₆ is H, or R₅ is H and R₆ is OH.

Preferably, compound (I) is characterized by a compound of the formula (II) 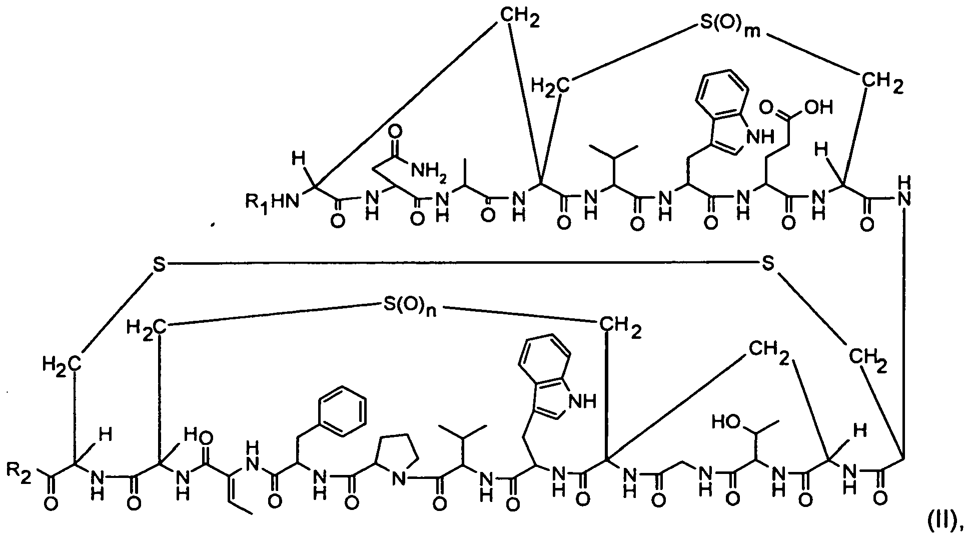 wherein R₁ is R₁' or a group 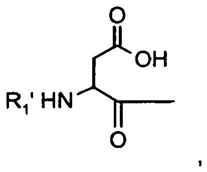 wherein R₁' is H, C(O)-(C₁-C₆)alkyl or C(O)-O-(C₁-C₆)alkyl, preferably H.

Further preferred, compound (I) is characterized by a compound of the formula (III) 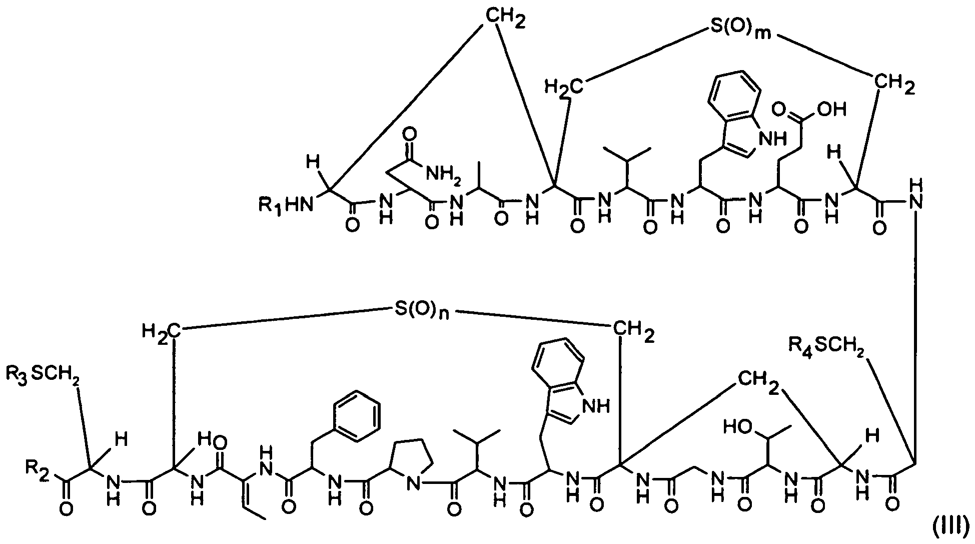 wherein
R₁ is R₁' or a group 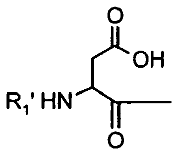 wherein R₁' is H, C(O)-(C₁-C₆)alkyl or C(O)-O-(C₁-C₆)alkyl, preferably H;
R₂ is OH, NH₂, NH-(C₁-C₆)-alkyl, N[(C₁-C₆)-alkyl]₂, NH-(C₁-C₄)-alkylene-phenyl or NH-(C₁-C₄)-alkylene-pyridyl, preferably R₂ is H; and
R₃ and R₄ are independently from each other H, (C₁-C₆)alkyl or (C₁-C₄)-alkylene-C(O)NH₂.

Compounds of the formulae (II) and (III) wherein R₁ is R₁' are subsequently named Labyrinthopeptins A1.

Compounds of the formulae (II) and (III) wherein R₁ is a group 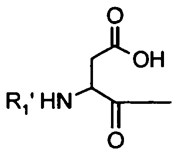 are subsequently named Labyrinthopeptins A3.

Further preferred, compound (I) is characterized by a compound of the formula (IV) 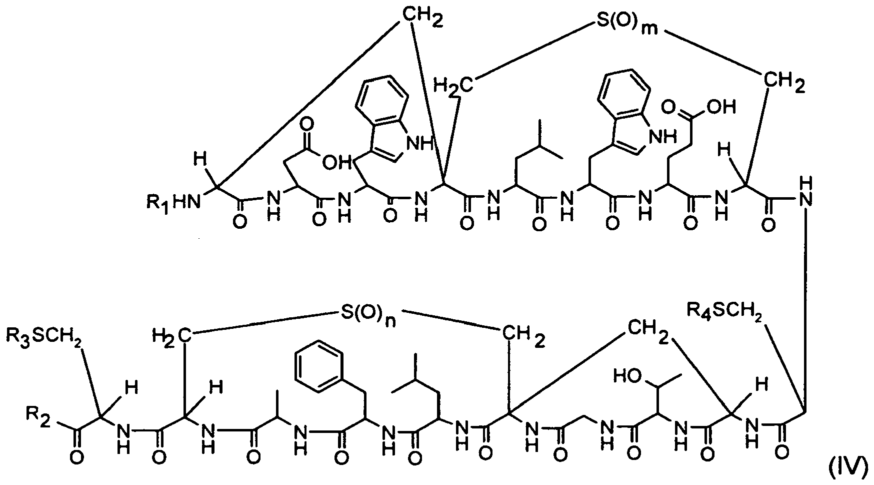 wherein
R₁ is H, C(O)-(C₁-C₆)alkyl or C(O)-O-(C₁-C₆)alkyl, and
R₂ is OH, NH₂, NH-(C₁-C₆)-alkyl, N[(C₁-C₆)-alkyl]₂, NH-(C₁-C₄)-alkylene-phenyl or NH-(C₁-C₄)-alkylene-pyridyl, and
R₃ and R₄ are independently from each other H, (C₁-C₆)alkyl or (C₁-C₄)-alkylene-C(O)NH₂.

Compounds of the formula (IV) are named Labyrinthopeptins A2.

Preferably, in the compounds of the formula (I), m and n are both 0, or m and n are both 2, or m is 0 and n is 2, or m is 2 and n is 0. Most preferred, m and n are both 0.

Unless otherwise indicated, the chiral centers in the compounds of the formula (I) can be present in the R configuration or in the S configuration. The invention relates both to the optically pure compounds and to stereoisomeric mixtures, such as enantiomeric mixtures and diastereomeric mixtures.

Physiologically tolerated salts of compounds of the formula (I) are understood as being both their organic salts and their inorganic salts, as are described in Remington's Pharmaceutical Sciences (17th edition, page 1418 (1985)). Because of their physical and chemical stability and their solubility, sodium, potassium, calcium and ammonium salts are preferred, inter alia, for acid groups; salts of hydrochloric acid, sulfuric acid or phosphoric acid, or of carboxylic acids or sulfonic acids, such as acetic acid, citric acid, benzoic acid, maleic acid, fumaric acid, tartaric acid and p-toluenesulfonic acid, are preferred, inter alia, for basic groups.

More preferred, the compounds of the formulae (I) to (IV) are characterized by the stereochemistry as shown for a compound of the formula (V), that is a compound of the formula (I), wherein
{A} is 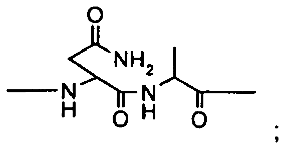
{B} is 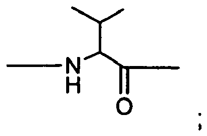
{C} is 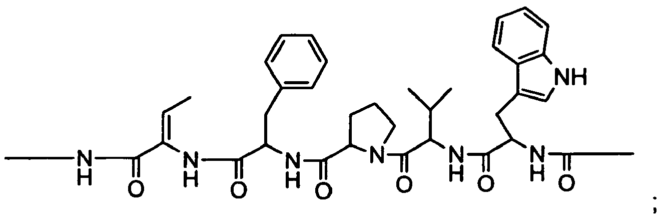
R₁ is 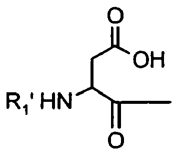 wherein R₁' is H;
R₂ are H;
R₃ and R₄ together with the S atoms to which they are attached form a disulfide group S-S;
R₅ is H;
R₆ is OH; and
m and n are 0: 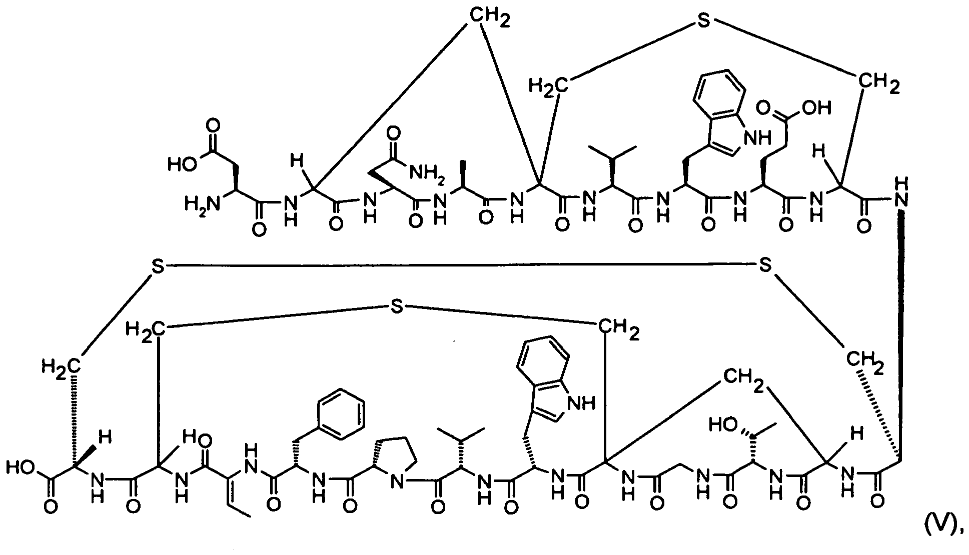 most preferred, as described in the formula (VI)

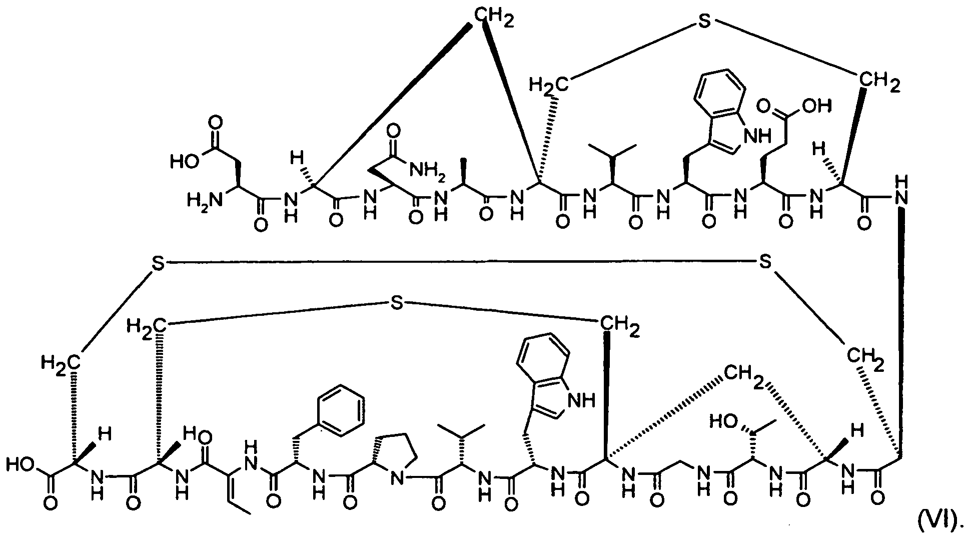

A further embodiment of the present invention is a compound of the formula (I), characterized by the formula (VII) 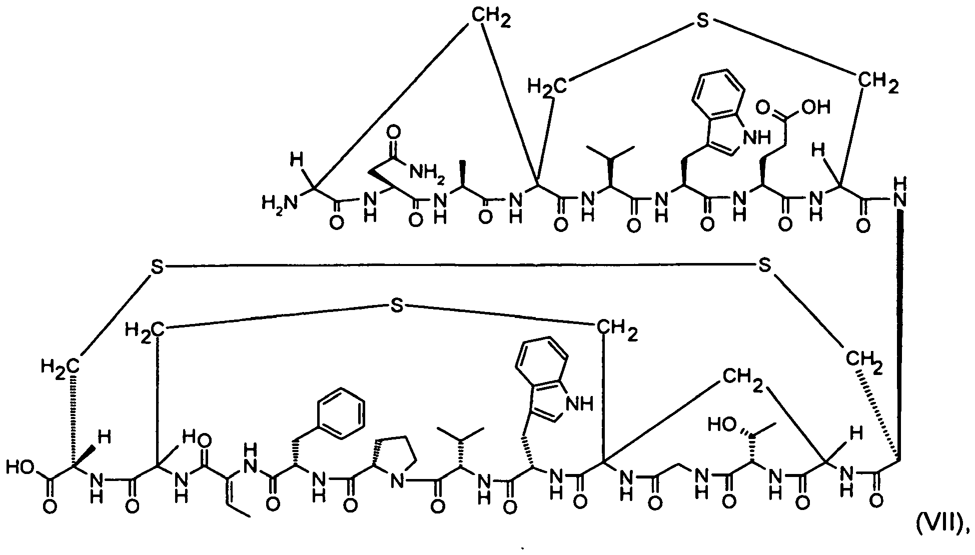 preferably by formula (VIII) 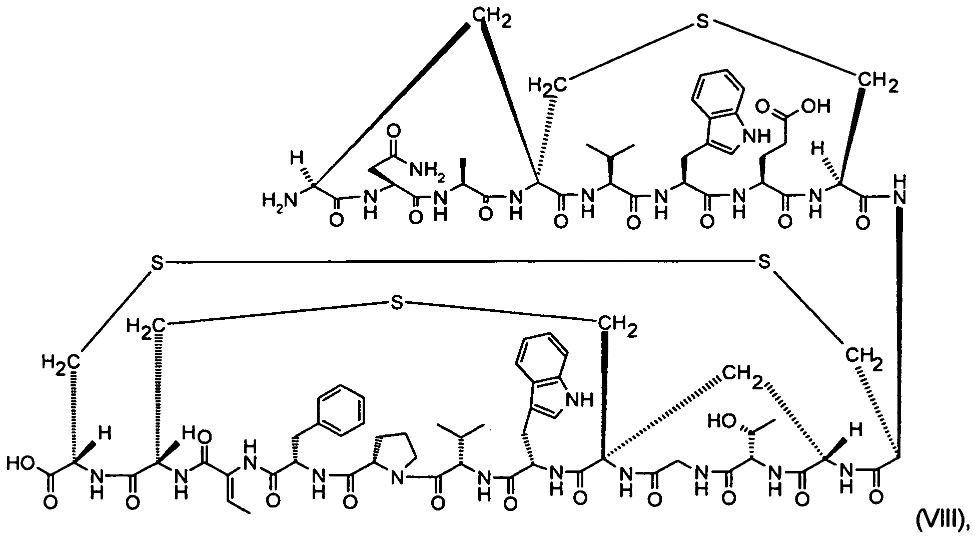 wherein the formulae (V) and (VI) refer to Labyrinthopeptin A3, and the formulae (VII) and (VIII) refer to Labyrinthopeptin A1.

For a further characterization of the compounds of the present invention, the peptide residues were converted back to their probable precursors from ribosomal peptide synthesis. The alpha,alpha-disubstituted amino acids in residues 4 and 13 are without precedence in the literature. The said amino acids may be described as a Ser residue, where the hydroxyl group at the beta-position is substituted, as shown below for the compounds of the formulae (II) and (III):

In a preceding European patent application for Labyrinthopeptin A2 (EP06020980.6), the following ribosomal precursor was assumed in the absence of knowledge on the biosynthesis:

On the basis of new insights in the biosynthesis of Labyrinthopeptins (see below), the biosynthetic precursor for compounds of the formula (IV) is described as follows:

The invention also relates to a process for preparing a compound of the formula (I) according to claim 1 comprising
a) fermenting the strain *Actinomadura namibiensis* (DSM 6313), or one of its variants and/or mutants, under suitable conditions in a culture medium until one or more of the compounds of the formula (I) accrue(s) in the culture medium,
b) isolating a compound of the formula (I) from the culture medium, and
c) derivatizing, where appropriate, the compound isolated in step b) and/or, where appropriate, converting the compound isolated in step b) or the derivative of compound isolated in step b) into a physiologically tolerated salt.

Preferably, the compound isolated in step b) is characterized by formula (II) wherein m and n are both 0,
R₁ is R₁' or a group 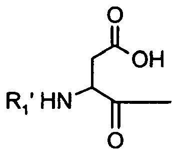 wherein R₁' is H, and
R₂ is OH.

Further preferred, the compound isolated in step b) is Labyrinthopeptin A2 which subsequently derivatized in step c) to a compound of the formula (IV) wherein m and n are both 0,
R₁ is H,
R₂ is OH, and
R₃ and R₄ are independently of each other H, (C₁-C₆)alkyl, (C₁-C₆)alkylene-C(O)NH₂, (C₁-C₆)alkylene-C(O)NH(C₁-C₄)alkyl or (C₁-C₆)alkylene-C(O)N[(C₁-C₄)alkyl]₂.

The culture medium is a nutrient solution or a solid medium containing at least one customary carbon source and at least one nitrogen source as well as one or more customary inorganic salts.

The process according to the invention can be used for fermenting on a laboratory scale (milliliter to liter scale) and for fermenting on an industrial scale (cubic meter scale).

Suitable carbon sources for the fermentation are assimilable carbohydrates and sugar alcohols, such as glucose, lactose, sucrose or D-mannitol, as well as carbohydrate- containing natural products, such as malt extract or yeast extract. Examples of nitrogen-containing nutrients are amino acids; peptides and proteins and also their breakdown products, for example casein, peptones or tryptones; meat extracts; yeast extracts; gluten; ground seeds, for example from corn, wheat, beans, soya or the cotton plant; distillation residues from producing alcohol; meat meals; yeast extracts; ammonium salts; nitrates. Preference is given to the nitrogen source being one or more peptide(s) which has/have been obtained synthetically or biosynthetically. Examples of inorganic salts are chlorides, carbonates, sulfates or phosphates of the alkali metals, the alkaline earth metals, iron, zinc, cobalt and manganese. Examples of trace elements are cobalt and manganese.

Conditions which are especially suitable for forming the Labyrinthopeptins according to the invention are as follows: from 0.05 to 5%, preferably from 0.1 to 2.5%, yeast extract; from 0.2 to 5.0%, preferably from 0.1 to 2%, casitone; from 0.02 to 1.0%, preferably from 0.05 to 0.5%, CaCl₂ × 2 H₂O; from 0.02 to 1.5%, preferably from 0.05 to 0.7%, MgSO₄ × 7 H₂O and from 0.00001% to 0.001% cyanocobalamin. The percentage values which are given are in each case based on the weight of the total nutrient solution.

The microorganism is cultured aerobically, that is, for example, submerged while being shaken or stirred in shaking flasks or fermenters, or on solid medium, where appropriate while air or oxygen is being passed in. The microorganism can be cultured in a temperature range of from about 18 to 35°C, preferably at from about 20 to 32°C, in particular at from 27 to 30°C. The pH range should be between 4 and 10, preferably between 6.5 and 7.5. The microorganism is generally cultured under these conditions for a period of from 2 to 10 days, preferably of from 72 to 168 hours. The microorganism is advantageously cultured in several steps, i.e. one or more preliminary cultures are initially prepared in a liquid nutrient medium, with these preliminary cultures then being inoculated into the actual production medium, i.e. the main culture, for example in a ratio by volume of from 1:10 to 1:100. The preliminary culture is obtained, for example, by inoculating the strain, in the form of vegetative cells or spores, into a nutrient solution and allowing it to grow for from about 20 to 120 hours, preferably for from 48 to 96 hours. Vegetative cells and/or spores can be obtained, for example, by allowing the strain to grow for from about 1 to 15 days, preferably for from 4 to 10 days, on a solid or liquid nutrient substrate, for example yeast agar.

The Labyrinthopeptin derivatives can be isolated and purified from the culture medium using known methods and taking account of the chemical, physical and biological properties of the natural substances. HPLC was used to test the concentrations of the respective Labyrinthopeptin derivatives in the culture medium or in the individual isolation steps, with the quantity of the substance formed expediently being compared with a calibration solution.

For the isolation, the culture broth or the culture together with the solid medium is optionally lyophilized, and the Labyrinthopeptin derivatives are extracted from the lyophilizate using an organic solvent or a mixture of water and an organic solvent, preferably containing 50-90% organic solvent. Examples of organic solvents are methanol and 2-propanol. The organic solvent phase contains the natural substances according to the invention; it is concentrated, where appropriate, in vacuo and subjected to further purification.

The further purification of one or more compounds according to the invention is effected by chromatography on suitable materials, preferably, for example, on molecular sieves, on silica gel, on aluminum oxide, on ion exchangers or on adsorber resins or on reversed phases (RPs). This chromatography is used to separate the Labyrinthopeptin derivatives. The Labyrinthopeptin derivatives are chromatographed using buffered, basic or acidified aqueous solutions or mixtures of aqueous and organic solutions.

Mixtures of aqueous or organic solutions are understood as being all water-miscible organic solvents, preferably methanol, 2-propanol or acetonitrile, at a concentration of from 5 to 99% organic solvent, preferably from 5 to 50% organic solvent, or else all buffered aqueous solutions which are miscible with organic solvents. The buffers which are to be used are the same as specified above.

The Labyrinthopeptin derivatives are separated, on the basis of their differing polarities, by means of reversed phase chromatography, for example on MCI (adsorber resin, Mitsubishi, Japan) or Amberlite XAD (TOSOHAAS), or on other hydrophobic materials, for example on RP-8 or RP-18 phases. In addition, the separation can be effected by means of normal-phase chromatography, for example on silica gel, aluminum oxide and the like.

Buffered, basic or acidified aqueous solutions are understood as being, for example, water, phosphate buffer, ammonium acetate and citrate buffer at a concentration of up to 0.5 M, as well as formic acid, acetic acid, trifluoroacetic acid, ammonia and triethylamine, or all commercially available acids and bases known to the skilled person, preferably at a concentration of up to 1%. In the case of buffered aqueous solutions, particular preference is given to 0.1% ammonium acetate.

The chromatography can be carried out using a gradient which began with 100% water and ended with 100% organic solvent; the chromatography was preferably run with a linear gradient of from 5 to 95% acetonitrile.

Alternatively, it is also possible to carry out a gel chromatography or chromatography on hydrophobic phases. The gel chromatography can e.g. be carried out on polyacrylamide gels or copolymer gels. The sequence of the abovementioned chromatographic steps can be reversed.

Insofar as Labyrinthopeptins are present as stereoisomers, they can be separated using known methods, for example by means of separation using a chiral column.

The derivatization of the OH group to an ester or ether derivative is effected using methods which are known per se (J. March, Advanced Organic Chemistry, John Wiley & Sons, 4th edition, 1992), for example by means of reaction with an acid anhydride or by reaction with an di-alkyl carbonate or di-alkyl sulfate. Derivatization of the COOH group to an ester or amid derivative is effected using methods which are known per se (J. March, Advanced Organic Chemistry, John Wiley & Sons, 4th edition, 1992), for example by means of reaction with ammonia to the respective CONH₂ group, or with an optionally activated alkyl compound to the respective alkyl ester. Oxidation of -CH₂-S-CH₂- groups to a -CH₂-S(O)-CH₂- or a -CH₂-S(O)₂-CH₂- group can be achieved upon exposing the respective Labyrinthopeptin derivative to oxygen or air. Reduction of disulfides, optionally followed by alkylation of free SH groups, is effected using methods which are known per se (A. Henschen, Analysis of cyst(e)ine residues, disulfide bridges, and sulfhydryl groups in proteins, in: B. Wittmann-Liebold, J. Salnikov, V.A. Erdman (Eds.), Advanced Methods in Protein Microsequence Analysis, Springer, Berlin, 1986, pp. 244-255), for example the reduction by means of dithiothreitol, and the alkylation using alkyl iodides. Sulfide reduction to a compound of the formula (I) wherein R₃ and R₄ are H, (C₁-C₆)alkyl, (C₁-C₆)alkylene-C(O)NH₂, (C₁-C₆)alkylene-C(O)NH(C₁-C₄)alkyl or (C₁-C₆)alkylene-C(O)N[(C₁-C₄)alkyl]₂ can be achieved by reacting a compound of the formula (I) wherein R₃ and R₄ form a disulfide group S-S together with the S atoms to which they are attached with an (C₁-C₆)alkylhalogenide or halogen-(C₁-C₆)alkylene-C(O)NH₂, halogen-(C₁-C₆)alkylene-C(O)NH(C₁-C₄)alkyl or halogen-(C₁-C₆)alkylene-C(O)N[(C₁-C₄)alkyl]₂ in the presence of dithiothreitol (general literature). Halogen is F, Cl, Br or I.

An isolate of the microorganism strain *Actinomadura namibiensis* was deposited by Hoechst AG, Frankfurt, Germany, under identification reference FH-A 1198 in the Deutsche Sammlung von Mikroorganismen und Zellkulturen [German Collection of Microorganisms and Cell Cultures] GmbH (DSMZ), Mascheroder Weg 1 B (as of 2008: Inhoffenstr. 7 B), 38124 Braunschweig, Germany, in accordance with the rules of the Budapest treaty, on 23.01.1991 under the following number: DSM 6313. Microorganism strain *Actinomadura namibiensis* is further described by Wink et al. in International Journal of Systematic and Evolutionary Microbiology 2003, 53, 721-724. Instead of the strain *Actinomadura namibiensis* (DSM 6313), it is also possible to use its mutants and/or variants which synthesize one or more of the compounds according to the invention.

A mutant is a microorganism in which one or more genes in the genome has/have been modified, with the gene, or the genes, which is/are responsible for the ability of the organism to produce the compound according to the invention remaining functional and heritable.

Such mutants can be produced, in a manner known per se, using physical means, for example irradiation, as with ultraviolet rays or X-rays, or chemical mutagens, such as ethyl methanesulfonate (EMS); 2-hydroxy-4-methoxybenzophenone (MOB) or N-methyl-N'-nitro-N-nitrosoguanidine (MNNG), or as described by Brock et al. in "Biology of Microorganisms", Prentice Hall, pages 238-247 (1984).

A variant is a phenotype of the microorganism. Microorganisms have the ability to adapt to their environment and therefore exhibit highly developed physiological flexibility. All the cells of the microorganism are involved in the phenotypic adaptation, with the nature of the change not being genetically conditioned and being reversible under altered conditions (H. Stolp, Microbial ecology: organism, habitats, activities. Cambridge University Press, Cambridge, GB, page 180, 1988).

Screening for mutants and/or variants which synthesize one or more of the compounds according to the invention is achieved by optionally lyophilizing the fermentation medium and extracting the lyophilizate or the fermentation broth with an organic solvent or a mixture of water and an organic solvent as defined above, and analyzing by means of HPLC or TLC or by testing the biological activity.

The fermentation conditions may be applied to *Actinomadura namibiensis* (DSM 6313) and for mutants and/or variants thereof.

A further embodiment of the present invention is the use of a compound of the formula (I), as defined above, for the treatment of bacterial infections, especially bacterial infections caused by Gram-positive bacteria, for the treatment of viral infections and/or for the treatment of pain, especially neuropathic pain or inflammatory triggered pain.

The above described medicament (also referred to as pharmaceutical preparation or pharmaceutical composition) contains an effective amount of at least one compound of the formula (I), in any stereochemical form, or a mixture of any stereochemical forms in any ratio, or a physiologically tolerable salt or chemical equivalent thereof, as described above, and at least one pharmaceutically acceptable carrier, preferably one or more pharmaceutically acceptable carrier substances (or vehicles) and/or additives (or excipients).

The medicament can be administered orally, for example in the form of pills, tablets, lacquered tablets, coated tablets, granules, hard and soft gelatine capsules, solutions, syrups, emulsions, suspensions or aerosol mixtures. Administration, however, can also be carried out rectally, for example in the form of suppositories, or parenterally, for example intravenously, intramuscularly or subcutaneously, in the form of injection solutions or infusion solutions, microcapsules, implants or rods, or percutaneously or topically, for example in the form of ointments, solutions or tinctures, or in other ways, for example in the form of aerosols or nasal sprays.

The medicaments according to the invention are prepared in a manner known per se and familiar to one skilled in the art, pharmaceutically acceptable inert inorganic and/or organic carrier substances and/or additives being used in addition to the compound(s) of the formula (I) in any stereochemical form, or a mixture of any stereochemical forms in any ratio, or a physiologically tolerable salt or chemical equivalent thereof, as described above. For the production of pills, tablets, coated tablets and hard gelatine capsules it is possible to use, for example, lactose, corn starch or derivatives thereof, talc, stearic acid or its salts, etc. Carrier substances for soft gelatine capsules and suppositories are, for example, fats, waxes, semisolid and liquid polyols, natural or hardened oils, etc. Suitable carrier substances for the production of solutions, for example injection solutions, or of emulsions or syrups are, for example, water, saline, alcohols, glycerol, polyols, sucrose, invert sugar, glucose, vegetable oils, etc. Suitable carrier substances for microcapsules, implants or rods are, for example, copolymers of glycolic acid and lactic acid. The pharmaceutical preparations normally contain about 0.5 to about 90 % by weight of a compound of the formula (I) and/or their physiologically acceptable salts and/or their prodrugs. The amount of the active ingredient of the formula (I) in any stereochemical form, or a mixture of any stereochemical forms in any ratio, or a physiologically tolerable salt or chemical equivalent thereof, as described above, in the medicaments normally is from about 0.5 to about 1000 mg, preferably from about 1 to about 500 mg.

In addition to the active ingredients of the formula (I) in any stereochemical form, or a mixture of any stereochemical forms in any ratio, or a physiologically tolerable salt or chemical equivalent thereof, as described above, and to carrier substances, the pharmaceutical preparations can contain one or more additives such as, for example, fillers, disintegrants, binders, lubricants, wetting agents, stabilizers, emulsifiers, preservatives, sweeteners, colorants, flavorings, aromatizers, thickeners, diluents, buffer substances, solvents, solubilizers, agents for achieving a depot effect, salts for altering the osmotic pressure, coating agents or antioxidants. They can also contain two or more compounds of the formula (I) in any stereochemical form, or a mixture of any stereochemical forms in any ratio, or a physiologically tolerable salt or chemical equivalent thereof. In case a pharmaceutical preparation contains two or more compounds of the formula (I), the selection of the individual compounds can aim at a specific overall pharmacological profile of the pharmaceutical preparation. For example, a highly potent compound with a shorter duration of action may be combined with a long-acting compound of lower potency. The flexibility permitted with respect to the choice of substituents in the compounds of the formula (I) allows a great deal of control over the biological and physico-chemical properties of the compounds and thus allows the selection of such desired compounds. Furthermore, in addition to at least one compound of the formula (I), the pharmaceutical preparations can also contain one or more other therapeutically or prophylactically active ingredients.

When using the compounds of the formula (I) the dose can vary within wide limits and, as is customary and is known to the physician, is to be suited to the individual conditions in each individual case. It depends, for example, on the specific compound employed, on the nature and severity of the disease to be treated, on the mode and the schedule of administration, or on whether an acute or chronic condition is treated or whether prophylaxis is carried out. An appropriate dosage can be established using clinical approaches well known in the medical art. In general, the daily dose for achieving the desired results in an adult weighing about 75 kg is from about 0.01 to about 100 mg/kg, preferably from about 0.1 to about 50 mg/kg, in particular from about 0.1 to about 10 mg/kg, (in each case in mg per kg of body weight). The daily dose can be divided, in particular in the case of the administration of relatively large amounts, into several, for example 2, 3 or 4, part administrations. As usual, depending on individual behaviour it may be necessary to deviate upwards or downwards from the daily dose indicated.

### Example 1: Preparation of a cryoculture of Actinomadura namibiensis (DSM 6313)

100 ml culture medium (10 g starch, 2 g yeast extract, 10 g glucose, 10 g glycerine, 2.5 g cornsteep powder, 2 g peptone, 1 g NaCl, 3g CaCO₃ in 1 I tap water, pH 7.2 before sterilization) were seeded with the strain *Actinomadura namibiensis* (DSM 6313) in a sterile 500 ml Erlenmeyer flask and incubated for 72 hours at 27°C and 120 rpm on a shaker. Subsequently, 1 ml of the culture and 1 ml sterile conservation solution (20 g glycerine, 10 g saccharose, 70 ml de-ionised water) were mixed and stored at -80°C. Alternatively, small pieces of a well-grown culture on agar were transferred into Cryotubes® (Vangard International) with 1.5 ml 50% sterile glycerine solution and stored at -196°C in liquid nitrogen.

### Example 2: Preparation of Labyrinthopeptins

A sterile 500 ml Erlenmeyer flask containing 100 ml of the culture medium described in Example 1 was seeded with a culture of *Actinomadura namibiensis* (DSM 6313) which was grown on an agar plate and was incubated at 27°C and 120 rpm on a shaker. After 72 hours, further Erlenmeyer flasks containing the same culture medium in the same amount were seeded with 2 ml of this pre-culture each and incubated under identical conditions for 168 hours. Alternatively, a 300 ml Erlenmeyer flask containing 100 ml of the culture medium described in Example 1 was seeded with a culture of *Actinomadura namibiensis* (DSM 6313) and incubated at 25°C and 180 rpm. After 72 hours, further Erlenmeyer flasks containing the same culture medium in the same amount were seeded with 5 ml of this pre-culture each and incubated under identical conditions for 168 hours.

### Example 3: Solid phase extraction of Labyrinthopeptins

After completion of a 40 L-fermentation of *Actinomadura namibiensis* (DSM 6313) the culture broth has been filtered. The culture filtrate (ca. 30 L) has been loaded onto a column (dimension: 160 x 200 mm) filled ca. 3 L of CHP-20P material. Compounds were eluted at a flow rate of 250 ml/min using a gradient from 5% to 95% of isopropanol in water. Fractions have been collected every 4 min over a period of 45 min. Fractions containing the Labyrinthopeptins have been pooled and freeze-dried (Fraction 8: MW = 2190 Da; Fraction 9: MW = 2190 and 2074 Da; Fraction 10-12: MW = 2074 Da).

### Example 4: Pre-purification of Labyrinthopeptin A1 using RP-18 Chromatography

Fraction 10-12 (670 mg) from Example 3 has been dissolved in 500 ml methanol and loaded onto a Phenomenex Luna® 10µ C18 (2) column (dimension: 50 mm x 250 mm) with a Phenomenex Luna® 10µ C18 (2) pre-column (dimension: 21.2 mm x 60 mm). Compounds were eluted with a gradient from 5% to 75% acetonitrile in water over a period of 40 min at a flow rate of 190 ml/min (buffer: 0.1 % ammonium acetate, pH 9.0, adjusted using a 30% aqueous ammonia solution). Fractions were collected every minute. Fractions 21-22 contained the desired Labyrinthopeptin (MW = 2074 Da). After freeze-drying, 322 mg crude product was obtained.

### Example 5: Final purification of Labyrinthopeptin A1

Fractions 21-22 from Example 4 (60 mg) have been dissolved in 50 ml methanol and loaded onto a Phenomenex Luna® 5µ C18 (2) Axia column (dimension: 30 mm x 100 mm) with a Waters XTerra® Prep MS C18 10µ pre-column (dimension: 19 x 10 mm). Compounds were eluted with a gradient from 5% to 75% acetonitrile in water over a period of 40 min at a flow rate of 70 ml/min (buffer: 0.1 % ammonium acetate, pH 4.6, adjusted using aqueous acetic acid). The eluents have been collected in 10 ml-fractions using UV-triggering. Labyrinthopeptin-containing fractions (f. 9-12) have been pooled. After freeze-drying, 17 mg of Labyrinthopeptin A1 have been obtained.

### Example 6: Pre-purification of Labyrinthopeptin A3 using RP-18 chromatography

Fraction 8 (~ 850 mg) from example 3 has been dissolved in 500 ml methanol and loaded onto a Phenomenex Luna® 10µ C18 (2) column (dimension: 50 mm x 250 mm) with a Phenomenex Luna® 10µ C18 (2) pre-column (dimension: 21.2 mm x 60 mm). Compounds were eluted with a gradient from 5% to 75% acetonitrile in water over a period of 40 min (buffer: 0.1 % ammonium acetate, pH 7.0) at a flow rate of 190 ml/min. Fractions were collected every minute. Fraction 19 contained the desired Labyrinthopeptin (MW = 2190 Da). After freeze-drying, 48 mg crude product was obtained.

### Example 7: Final purification of Labyrinthopeptin A3

Fraction 19 from example 6 (48 mg) has been dissolved in 50 ml methanol and loaded onto a Phenomenex Luna® 5µ C18 (2) Axia column (dimension: 30 mm x 100 mm) with a Waters XTerra® Prep MS C18 10µ pre-column (dimension: 19 mm x 10 mm). Compounds were eluted with a gradient from 5% to 75% acetonitrile in water over a period of 40 min at a flow rate of 70 ml/min (buffer: 0.1 % ammonium acetate, pH 9.0, adjusted using a 30% aqueous ammonia solution). The eluents have been collected in fractions using UV-triggering. Labyrinthopeptin-containing fractions (F9-12) have been pooled. After freeze-drying, 12 mg of Labyrinthopeptin A3 have been obtained. Example 8: Characterisation of Labyrinthopeptins A1 and A3 by high performance liquid chromatography with diode-array and mass spectrometry detection (HPLC-DAD-MS)

Labyrinthopeptins A1 and A3 were analyzed on a Waters Acquity UPLC System with Sample Manager, Binary Solvent Manager and PDA (Photodiode Array Detector). As UPLC column a Waters Acquity UPLC BEH C18 (1.7µ; 2.1x100 mm) was used and eluted at a flow rate of 0.6 ml/min with a gradient of water:acetonitrile (9:1) within 15 min to 100 % acetonitrile, all solvents buffered with 6.5mM ammonium acetate to pH 4.6. UV spectra were recorded by the PDA detector at wavelengths between 200 and 600 nm. Mass spectra were recorded with a Bruker µTOF LC MS using an orthogonal electrospray ionisation, a sampling-rate of 0.5 Hz and a detection-limit of 150-1500 atomic mass units.

### Example 9: Characterisation of Labyrinthopeptins A1

Labyrinthopeptin A1 eluted at 5.46 min (PDA). The UV spectrum is featured by λₘₐₓ of 218 nm (sh) and 279 nm.

Doubly-charged molecular ions were observed at m/z (I): 1035.87 (4539), 1036.37 (5566), 1036.87 (4086), 1037.37 (2296), 1037.87 (1034) and 1038.37 (280) in the negative mode. In positive mode doubly-charged molecular ions of m/z (I): 1037.88 (2925), 1038.38 (3252), 1038.88 (2492), 1039.38 (1396), and 1039.88 (623) were observed.

Characterisation of Labyrinthopeptin A1 by high resolution ESI-FTICR-mass spectrometry: A solution of Labyrinthopeptin A1 in methanol (c = 0.2 mg/ml) was admitted through a syringe pump at a flow rate of 2 µl/min to a Bruker Apex III FTICR MS (7T magnet) equipped with an electrospray source. Spectra were recorded in the positive mode using an external calibration.

| | |
|---|---|
| m/z observed in Da (z=2, M+2Na⁺ ion) | 1059.8693 |
| Exact, mono-isotopic mass of neutral [M] | 2073.7592 |
| Theoretical mass [M] for C₉₂H₁₁₉N₂₃O₂₅S₄ | 2073.7630 |
| Molecular formula | C₉₂H₁₁₉N₂₃O₂₅S₄ |

### Example 10: Characterisation of Labyrinthopeptin A3

Labyrinthopeptin A3 eluted at 4.79 min (PDA). The UV spectrum is featured by λₘₐₓ of 218 nm (sh) and 274 nm (sh).

Doubly-charged molecular ions were observed at m/z (I): 1093.38 (1262), 1093.88 (1587), 1094.39 (1201), 1094.89 (686) and 1095.38 (195) in the negative mode. In positive mode, doubly-charged molecular ions of m/z (I): 1095.40 (365), 1095.91 (433) and 1096.41 (294) were observed.

Characterisation of Labyrinthopeptin A3 by high resolution ESI-FTICR-mass spectrometry (method as described in Example 9):

| | |
|---|---|
| m/z observed in Da (z=2, M+2Na⁺ ion) | 1117.3847 |
| Exact, mono-isotopic mass of neutral [M] | 2188.7900 |
| Theoretical mass [M] for C₉₆H₁₂₄N₂₄O₂₈S₄ | 2188.7900 |
| Molecular formula | C₉₆H₁₂₄N₂₄O₂₈S₄ |

### Example 11: Amino acid analysis of Labyrinthopeptin A1

Hydrolysis: Labyrinthopeptin A1 (0.05 mg) was hydrolyzed in nitrogen atmosphere with 6 N HCl, 5% phenole at 110°C for 24 h. The hydrolysate was dried in a stream of nitrogen.

Achiral GC-MS: The hydrolysate was heated with bis-(trimethylsilyl)trifluoroacetamide (BSTFA)/acetonitrile (1:1) at 150 °C for 4 h. For GC-MS experiments a DB5-fused-silica-capillary (I = 15 m × 0.25 µm fused silica coated with dimethyl-(5%-phenylmethyl)-polysiloxane, d_{f} = 0.10 µm; temperature programme: T = 65°/3'/6/280°C) was used.

Chiral GC-MS: The hydrolysate was esterified with 200 µl 2 N HCl in ethanol at 110 °C for 30 min and dried. Subsequently, the mixture was acylated with 25 µl trifluoroacetic acid anhydride (TFAA) in 100 µl dichloromethane at 110°C 10 min for and dried. For GC-MS a fused-silica-capillary was used (I = 22 m × 0.25 µm fused silica coated with chirasil-S-Val (Machery-Nagel), d_{f} = 0.13 µm; temperature programme: T = 55°/3'/3,2/180°C).

| | | |
|---|---|---|
| | | configuration |
| Amino acids | 1 Ala, 1 Thr, 1 Asx, 2 Cys, 1 Phe, 1 Glx, 2 Trp, 1 Gly, 2 Val, 1 Pro = 13 AS | all S-amino acids, except for 2 Cys in the R configuration |

### Example 12: Identification of the structural genes for Labyrinthopeptins A1 and A3

A cosmid bank of the microorganism *Actinomadura namibiensis* (DSM 6313) was generated by Agowa GmbH, Berlin, based on the pWEB-cosmid vector (Epicentre Biotechnologies, Madison, USA). Filters were prepared by RZPD GmbH, Berlin, applying a methodology described in: Zehetner & Schäfer, Methods Mol. Biol. 2001, 175, 169-188.

Based on the known structure of Labyrinthopeptin A2, elongated degenerated primers were deduced from the N-terminal and C-terminal end (Fw: 5'-CAGGAAACAGCTATGACCGAYTGGWSNYTNTGGG-3' (SEQ 10 NO: 4); Rev: 5'-TGTAAAACGACGGCCAGTRCANGANGCRAANARRC-3' (SEQ ID NO: 5); Dabard et al., Appl. Environ. Microbiol. 2001, 4111-4118.). The 5'-elongation of the primers was to enhance the expected PCR-product size for better detection and handling (PCR-conditions: 3 min 95 °C; 30 x (60 s 95 °C; 30 s 50 °C; 60 s 72 °C) 7 min 72 °C; Taq-polymerase). The PCR-product was gel-purified and cloned into the vector pDrive (Qiagen). Sequencing resulted in a 18 nucleotide length sequence from the middle of the A2 gene (AGTGCTGTAGCACGGGAA, SEQ ID NO: 6). Based on this 18 nucleotide long known sequence, a two-step PCR rendered to more sequence information. In the first step, a single-specific primer-PCR was performed with a degenerated reversed(rev)-primer of the C-terminal end of A2 (5'-RCARCANGCRAANARRCTTCC-3', SEQ ID NO: 7)and an unspecific forward(fw)-primer (5'-CACGGTACCTAGACTAGTGACCAAGTGCGCCGGTC-3', SEQ ID NO: 8) (PCR-conditions: 3 min 95°C; 10 x (45 s 95°C; 45 s 38°C; 3.5 min 72°C); 30 x (45 s 95°C; 45 s 52°C; 3.5 min 72°C) 5 min 72°C; Taq-polymerase). After an exonuclease-I digest in order to digest the primers (5 µl PCR-sample + 0.5 µl exonuclease-I (20U/µl); 15 min 37°C; 15 min 80°C heat inactivation), the PCR-sample was used as template for a second PCR (PCR-conditions: 3 min 95°C; 30 x (45 s 95°C; 45 s 56°C; 3,5 min 72°C) 5 min 72°C; Taq-polymerase). The second PCR was performed in a nested-PCR manner with a primer pair consisting of the unspecific fw-primer from the first PCR and a specific rev-primer, including the known 18 nucleotides (5'-CTTCCCGTGCTACAGCACTCCC-3', SEQ ID NO: 9). The 0.4 kbp product was gel-purified and cloned into pDrive. Sequencing showed the expected amino acid sequence of the C-terminal end of A2. Out of this 0.4 kbp sequence, a Dig-labelled probe was constructed by PCR (Fw: 5'-ATGGACCTCGCCACGGGCTC-3', SEQ ID NO: 10; 5'-CTTCCCGTGCTACAGCACTCCC-3', SEQ ID NO: 11). This Dig-labelled probe was used to screen the filters by hybridization and detection via anti-Dig-antibody labeled with alkaline phosphatase. In this manner, one positive cosmid was obtained and sequenced.

Sequence data were analyzed by local blast and frameplot. The analysis yielded the following open reading frame (orf) that included the structural gene of Labyrinthopeptin A2:

Within this orf, the following sequence represents the structural gene of prepro-Labyrinthopeptin A2 (leader sequence followed by propeptide-encoding sequence followed by stop-codon TGA):

Translation of the DNA sequence as shown in SEQ ID NO: 13 gave the following amino acid sequence of prepro-Labyrinthopeptin A2 (SEQ ID NO: 14) and of pro-Labyrinthopeptin A2 (SEQ ID NO: 15):
MASILELQNLDVEHARGENR SDWSLWECCSTGSLFACC (SEQ ID NO: 14)
SDWSLWECCSTGSLFACC (SEQ ID NO: 15)

The propeptide sequence is transformed into Labyrinthopeptin A2 by posttranslational modifications by enzymes of the microorganism *Actinomadura namibiensis* (DSM 6313).

Example 13: Structure determination of Labyrinthopeptins A1 and A3:

The upstream region of the A2 gene displays another small orf with high homology to the structural gene of Labyrinthopeptin A2. This open reading frame (orf) included the structural gene of Labyrinthopeptin A1 and A3. The orf for Labyrinthopeptin A1 has the following gene sequence:

Within this orf, the following sequence represents the structural gene of prepro-Labyrinthopeptins A1 and A3 (leader sequence followed by propeptide-encoding sequence followed by stop-codon TGA):

Translation of the DNA sequence as shown in SEQ ID NO: 17 gave the following amino acid sequence of prepro-Labyrinthopeptin A1 (SEQ ID NO: 18) and of pro-Labyrinthopeptin A1 (SEQ ID NO: 19):
MASILELQDLEVERASSAADSNASVWECCSTGSWVPFTCC (SEQ ID NO: 18)
SNASVWECCSTGSWVPFTCC (SEQ ID NO: 19)

This amino acid sequence was in agreement with the expected amino acid composition of Labyrinthopeptin A1 based on results of amino acid- and MS-analysis of Labyrinthopeptin A1 (vide supra). Posttranslational modifications of side chains were deduced that are analogous to those of Labyrinthopeptin A2. The stereochemistry of amino acids has been taken from the amino acid analysis (Example 11). Finally, a dehydration of the threonine (Thr) residue to give dehydrobutyric acid was deduced to match the empirical molecular formula calculated from high-resolution MS. On the basis of the stereochemistry of the posttranslationally modified amino acid of the analogous Labyrinthopeptin A2, formula (VIII) is derived for Labyrinthopeptin A1.

Previous mass analysis suggested an Asp as the difference between Labyrinthopeptins A1 and A3. This was confirmed by the coded sequence, which included an Asp in position -1 ahead the protease cleavage side of Labyrinthopeptin A1. Under the assumption that that Labyrinthopeptins A1 and A3 are encoded by the same gene, differing only in the protease cleavage of the leader sequence, the additional Asp is at the N-terminus of Labyrinthopeptin A3. In this manner, formula (V) was derived for Labyrinthopeptin A3. On the basis of the stereochemistry of the posttranslationally modified amino acid of the analogous Labyrinthopeptin A2, formula (VI) is derived for Labyrinthopeptin A3.

### Example 14: Cleavage of the disulfide-bridge of Labyrinthopeptin A1 and subsequent alkylation with methyliodide

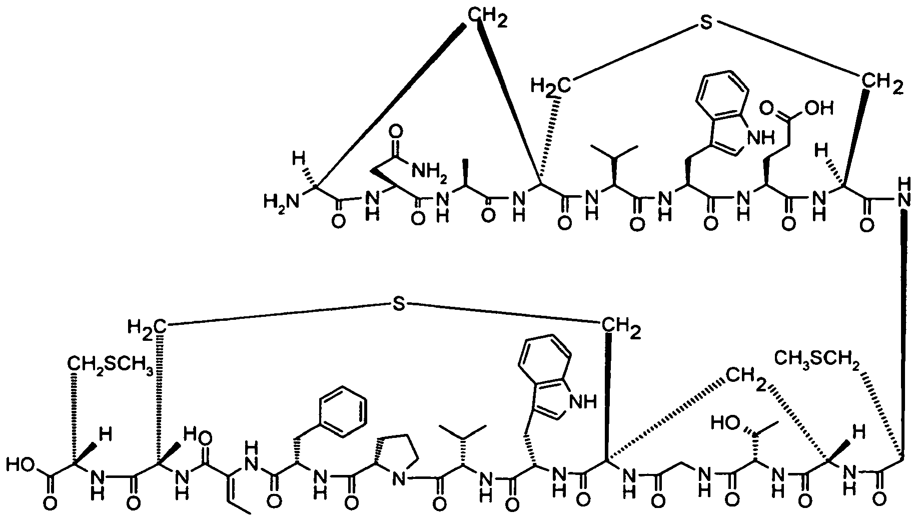

Labyrinthopeptin A1 (50 mg, 0.024 mmol) was dissolved in methanol (3 ml) and a dithiothreitol solution was added at room temperature (1 ml, freshly prepared from 75 mg dithiothreitol in a solution of 40 mg NaHCO₃ in 1 ml water). The mixture was stirred for 1 h at 60 °C. Afterwards it was cooled down to room temperature and methyliodide (50 µl, 0.80 mmol) was added. After 4 h at room temperature the mixture was filtered and purified by reversed phase HPLC using a Phenomenex Luna® Axia 5 µm C18 (2) column (dimension: 100 mm x 30 mm) with a Waters XTerra® Prep MS C18 10 µm pre-column (dimension: 19 mm x 10 mm). The gradient was running from 5% to 95% acetonitrile in water within 30 minutes (buffer: pH 2.0, adjusted with formic acid). The flow was 60 ml/min and the peaks were fractionated by UV. Fractions 12 and 13 were combined and yielded 23.1 mg (45.5 %) of the desired compound after lyophilization. The product was characterized by UV spectroscopy and mass spectrometry (Bruker Daltonics MicroTof).
RTₘᵢₙ = 5.46 min (PDA; LC-method as in Example 8)
UV (λₘₐₓ): 217 nm (sh), 279 nm
ESI-MS (neg): [M-2H]²⁻ = 1050.894
Experimental neutral monoisotopic mass, [M] = 2103.802
Neutral monoisotopic mass calculated for C₉₄H₁₂₅N₂₃O₂₅S₄: 2103.810
Molecular formula: C₉₄H₁₂₅N₂₃O₂₅S₄
Chemical molecular weight = 2105.44.

### Example 15: Cleavage of the disulfide-bridge of Labyrinthopeptin A1 and subsequent alkylation with iodo-acetamide

Labyrinthopeptin A1 (50 mg, 0.024 mmol) was dissolved in methanol (3 ml) and a dithiothreitol solution was added at room temperature (1 ml, freshly prepared from 70 mg dithiothreitol in a solution of 40 mg NaHCO₃ in 1 ml water). The mixture was stirred for 1 h at 60°C. Afterwards it was cooled down to room temperature and iodo-acetamide (40 mg, 0.216 mmol) was added. The mixture was stirred over night at room temperature. The solution was filtered and purified by reversed-phase HPLC using a Phenomenex Luna® Axia 5 µm C18 (2) column (dimension: 100 mm x 30 mm) with a Waters Terra® Prep MS C18 10 µm pre-column (dimension: 19 mm x 10 mm). The gradient was running from 5% to 95% acetonitrile in water within 30 minutes (buffer: 0.1% ammonium acetate, pH 4.6, adjusted with acetic acid). The flow was 60 ml/min and the peaks were fractionated by UV. The following compounds were obtained:
Bis-acetamided Labyrinthopeptin A1: 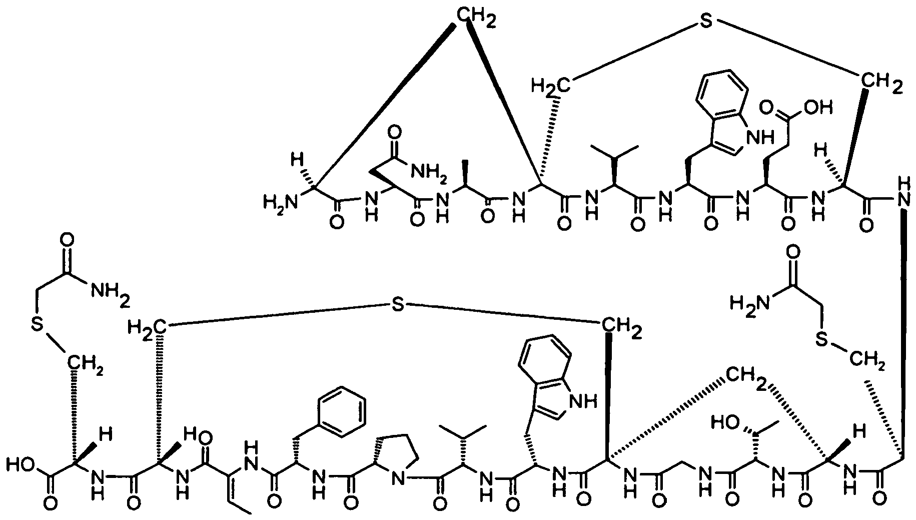 Fractions 7 and 8 were combined and yielded 13.3 mg (25.2 %) of the desired compound after lyophilization. The product was characterized by UV spectroscopy and mass spectrometry (Bruker Daltonics MicroTof).
   RTₘᵢₙ = 5.09 min (PDA; LC-method as in Example 8)
   UV (λₘₐₓ): 218 nm (sh), 280 nm
   ESI-MS (neg): [M-2H]²⁻ = 1093.9022
   Experimental neutral monoisotopic mass, [M] = 2189.819
   Neutral monoisotopic mass calculated for C₉₆H₁₂₇N₂₅O₂₇S₄: 2189.822
   Molecular formula: C₉₆H₁₂₇N₂₅O₂₇S₄
   Chemical molecular weight = 2191.49.
Mono-acetamided Labyrinthopeptin A1: 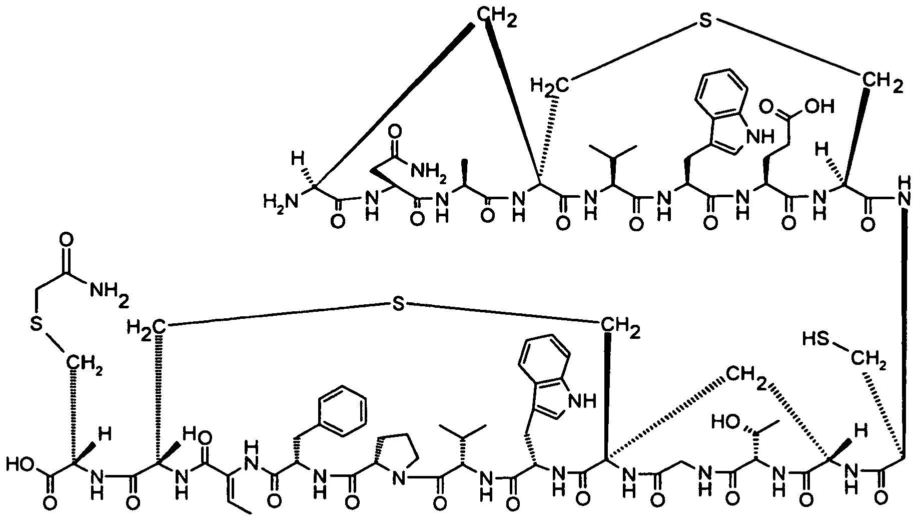 Fractions 10 and 11 were combined and yielded 5.3 mg (10.3 %) of the desired compound after lyophilization. The product was characterized by UV spectroscopy and mass spectrometry (Bruker Daltonics MicroTof).
   RTₘᵢₙ = 5.31 min (PDA; LC-method as in Example 8)
   UV (λₘₐₓ): 217 nm (sh), 280 nm
   ESI-MS (neg): [M-2H]²⁻ = 1065.390
   Experimental neutral monoisotopic mass, [M] = 2132.794
   Neutral monoisotopic mass calculated for C₉₄H₁₂₄N₂₄O₂₆S₄: 2132.800
   Molecular formula: C₉₄H₁₂₄N₂₄O₂₆S₄
   Chemical molecular weight = 2134.44.

### Example 16: Synthesis of a Boc-protected Labyrinthopeptin A1

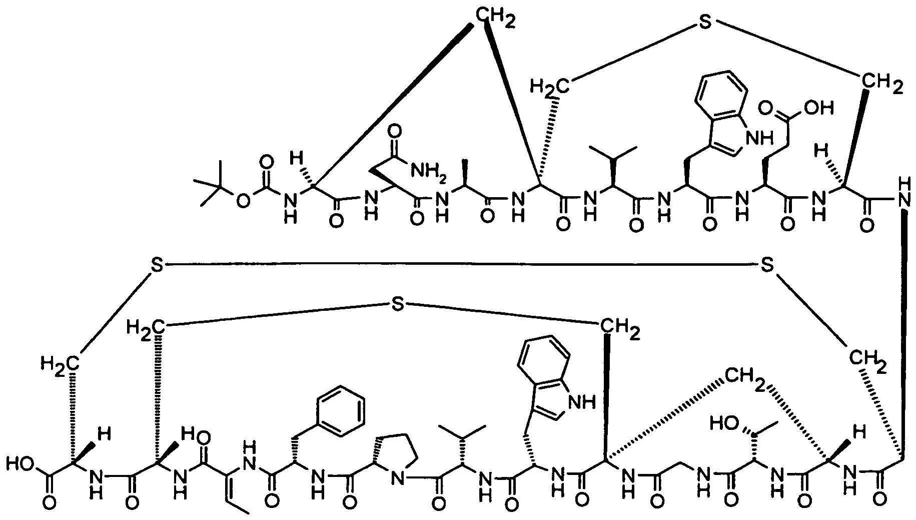

To a solution of Labyrinthopeptin A1 (50 mg, 0.024 mmol) in dimethylformamide (3 ml), di-tert-butyl-dicarbonate (11 mg, 0.048 mmol) and n-ethyldiisopropylamine (6 mg, 0.048 mmol) were added at room temperature. The mixture was stirred for 2 h at room temperature. Afterwards it was purified by reversed-phase HPLC using a Phenomenex Luna® Axia 5 µm C18 (2) column (dimension: 100 mm x 30 mm) with a Waters XTerra® Prep MS C18 10 µm pre-column (dimension: 19 mm x 10 mm). The gradient was running from 5% to 95% acetonitrile in water within 30 minutes (buffer: 0.1% ammonium acetate, pH 7.0). The flow was 60 ml/min and the peaks were fractionated by UV. Fractions 4-7 were combined and yielded 21.4 mg (40.8%) of the desired compound after lyophilization. The product was characterized by UV spectroscopy and mass spectrometry (Bruker Daltonics MicroTof).
RTₘᵢₙ = 5.30 min (PDA; LC-method as in Example 8)
UV (λₘₐₓ): 219 nm (sh), 278 nm
ESI-MS (neg): [M-2H]²⁻ = 1085.895
Experimental neutral monoisotopic mass, [M] = 2173.805
Neutral monoisotopic mass calculated for C₉₇H₁₂₇N₂₃O₂₇S₄: 2173.815
Molecular formula: C₉₇H₁₂₇N₂₃O₂₇S₄
Chemical molecular weight = 2174.49.

### Example 17: Benzyl derivatives of Labyrinthopeptin A1

To a solution of Labyrinthopeptin A1 (50 mg, 0.024 mmol) in dimethylformamide (2 ml), di-tert-butyl-dicarbonate (10 mg, 0.046 mmol) and n-ethyldiisopropylamine (7 mg, 0.054 mmol) were added at room temperature. After 1 h at room temperature, Labyrinthopeptin A1 was completely disappeared. Benzylamine (6.8 mg, 0.063 mmol) and n-propyl phosphonic acid anhydride (T3P®, 50 µl, 0.072 mmol, 50 % in DMF) were added. The mixture was stirred for 2 h at room temperature. Afterwards it was purified by reversed-phase HPLC using a Phenomenex Luna® Axia 5 µm C18 (2) column (dimension: 100 mm x 30 mm) with a Waters XTerra® Prep MS C18 10 µm pre-column (dimension: 19 mm x 10 mm). The gradient was running from 5% to 95% acetonitrile in water within 30 minutes (buffer: 0.1 % ammonium acetate, pH 7.0). The flow was 60 ml/ min and the peaks were fractionated by UV (220 nm). The following two compounds were obtained:
Mono-benzyl derivative of Labyrinthopeptin A1: 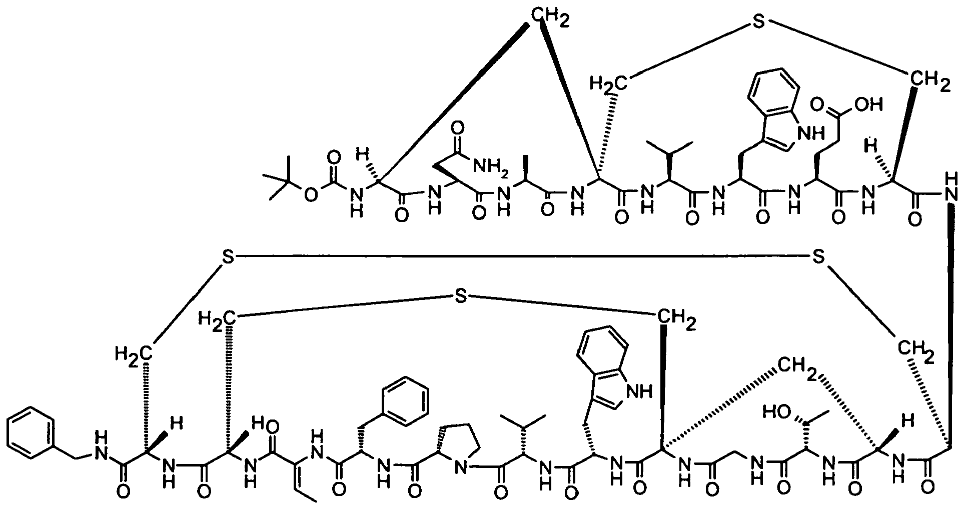 Fractions 7 and 8 were combined and yielded 10.4 mg (19.1 %) of the desired compound after lyophilization. The product was characterized by UV spectroscopy and mass spectrometry (Bruker Daltonics MicroTof).
   RTₘᵢₙ = 7.03 min (PDA; LC-method as in Example 8)
   UV (λₘₐₓ): 217 nm (sh), 275 nm
   ESI-MS (neg): [M-2H]²⁻ = 1130.427
   Experimental neutral monoisotopic mass, [M] = 2262.868
   Neutral monoisotopic mass calculated for C₁₀₄H₁₃₄N₂₄O₂₆S₄: 2262.878
   Molecular formula: C₁₀₄H₁₃₄N₂₄O₂₆S₄
   Chemical molecular weight = 2264.63.
Bis-benzyl derivative of Labyrinthopeptin A1: 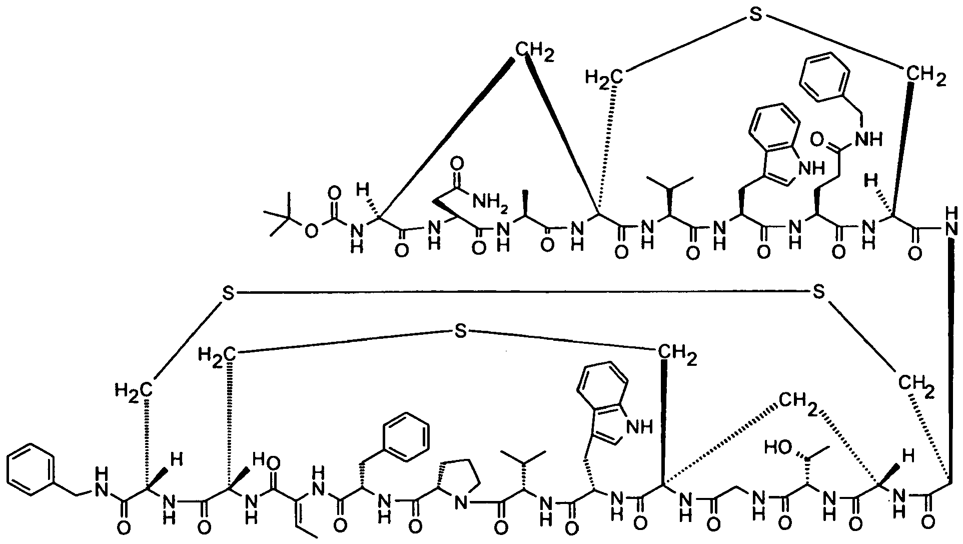 Fractions 13 and 14 were combined and yielded 9.9 mg (17.5 %) of the desired compound after lyophilization. The product was characterized by UV spectroscopy and mass spectrometry (Bruker Daltonics MicroTof).
   RTₘᵢₙ = 8.31 min (PDA; LC-method as in Example 8)
   UV (λₘₐₓ): 214 nm (sh), 276 nm
   ESI-MS (pos): [M+2(NH₄)]²⁺ = 1194.002
   Experimental neutral monoisotopic mass, [M] = 2351.937
   Neutral monoisotopic mass calculated for C₁₁₁H₁₄₁N₂₅O₂₅S₄: 2351.941
   Molecular formula: C₁₁₁H₁₄₁N₂₅O₂₅S₄
   Chemical molecular weight = 2353.77.

### Example 18: Acylation reactions at the N-terminus of Labyrinthopeptin A1

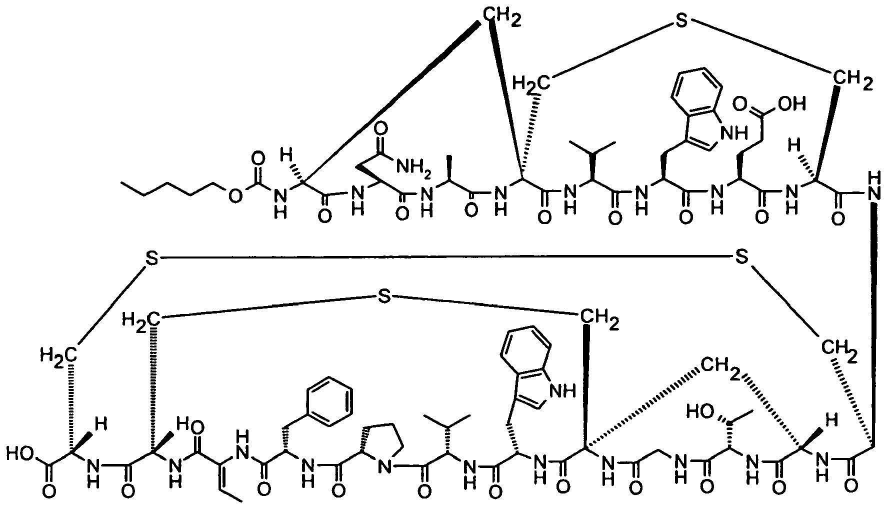

To a solution of 2-chloro-4,6-dimethoxy-1,3,5-triazine (CDMT, 5 mg, 0.028 mmol) in dimethylformamide (2 ml), n-methylmorpholine (8.6 mg, 0.085 mmol) was added at room temperature. After 1 h at room temperature n-hexane carboxylic acid (3.3 mg, 0.028 mmol) was added. After stirring the mixture for 30 minutes Labyrinthopeptin A1 (50 mg, 0.024 mmol) was added followed by stirring for 2 h at room temperature. The mixture was purified by reversed-phase HPLC using a Waters XBridge Shield® 5 µm C18 column (dimension: 100 mm x 30 mm) with a Waters XBridge Shield® C18 10 µm pre-column (dimension: 19 mm x 10 mm). The gradient was running from 5% to 95% acetonitrile in water within 30 minutes (buffer: 0.1 % ammonium acetate, pH 7.0). The flow was 60 ml/min and the peaks were fractionated by UV (220 nm). Fraction 39 yielded 2.0 mg (3.8%) of the desired compound after lyophilization. The product was characterized by UV spectroscopy and mass spectrometry (Bruker Daltonics MicroTof).
RTₘᵢₙ = 5.41 min (PDA; LC-method as in example 8)
UV (λₘₐₓ): 216 nm (sh), 266 nm
ESI-MS (neg): [M-2H]²⁻ = 1084.906
Experimental neutral monoisotopic mass, [M] = 2171.827
Neutral monoisotopic mass calculated for C₉₈H₁₂₉N₂₃O₂₆S₄: 2171.836
Molecular formula: C₉₈H₁₂₉N₂₃O₂₆S₄
Chemical molecular weight = 2173.52.

### Example 19: Antibacterial activity for Labyrinthopeptins and derivatives

The compounds were dissolved in water with 10 % MeOH to a final concentration of 1 mg/ml. For the bioassay sterile Nunc plates with a size of 24 x 24 cm were used. For one plate 200 ml of agar were used. The agar was cooled after autoclaving to 55°C and 2-4 ml of culture suspension of the test organism were added before plating. To each plate 64 filter plates with 6 mm in diameter were added.

To each filter 20 µl of the test solution were added and incubated for 1 to 3 days at 28 °C or 37 °C. The inhibition zone in mm was reported. For a detailed description of the methods, see Bauer et al., Amer. J. Clin. Pathol. 1966, 45, 493-496; Müller & Melchinger, Methoden in der Mikrobiologie, Franckhsche Verlagshandlung, Stuttgart (1964); Mueller & Hinton, Proc. Soc. Expt. Biol. Med. 1941, 48, 330-333.

| Tested compound | Streptomyces murinus (DSM 40091) 28°C | Bacillus subtilis (ATCC 6633) 37°C |
|---|---|---|
| Labyrinthopeptin A1, Ex. 7 | 13 | 16,5 |
| Labyrinthopeptin A1 derivative, Ex. 16 | 0 | 9 |
| Labyrinthopeptin A1 derivative, Ex. 18 | 7 | 8 |
| Labyrinthopeptin A1 Bis-benzyl derivative, Ex. 17 | 7 | 9 |
| Tetracycline (control substance, 1 mg/ml) | 24 | 32 |

### Example 20: Neuropathic pain activity

Labyrinthopeptin A1 was studied in the spared nerve injury (SNI) mouse model of neuropathic pain in order to proof the activity on tactile allodynia. Under general anaesthesia, the two major branches of the sciatic nerve in adult male C57B6 mice (weight: 22.8g +/- 0.35 SEM) have been ligated and transsected, with the sural nerve left intact. Tactile allodynia has been determined with the automatic von Frey test: using a dump needle stick, the plantar skin of hind paws was exposed to a pressure stimulus of increasing intensity up to 5 g. The force in grams at which the animal responded with hindpaw withdrawal was used as a read-out for tactile allodynia. The study was performed 7 days after nerve lesion over 6 hours with an additional measurement after 24 hours. Within two days after nerve transsection, tactile allodynia developed completely and remained stable over at least two weeks. The compound was administered intravenous as a single application (3 mg/kg). As a vehicle for the intravenous application was the 1:1:18 (ethanol:solutol: phosphate buffered saline) vehicle chosen.

Paw withdrawal threshold (PWT) measurements have been used to calculate significant treatment effects, and for AUC calculations over a reference time period (6 hours) and subsequent % benefit calculations. For the statistical analysis the PWT values of the ipsilateral hind paws were used in two ways: first, with a 2-way ANOVA based on the PWT values for specific times (within a period of 24 hours) and second with a 1-way ANOVA on non-transformed delta AUC values |AUC1-6hour|.

Two-way analysis of variance with repeated measures (Repeated factor: TIME, Analysis variable: PWT) followed by a Complementary Analysis (Effect of factor GROUP for each level of factor TIME (Winer analysis), Analysis variable: PWT) and a subsequent Dunnett's test for factor TREATMENT for each level of factor TIME (Two sided comparison vs level VEHICLE) revealed highly significant differences from the vehicle group from 1 to 6 hours after intravenous application for each compound. The effect was gone 24 hours after application. 1-way ANOVA using delta |AUC1-6hour| values revealed a p value of p <0.0001. Dunnett analysis and gave significant treatment effects for Labyrinthopeptin A1. The percent benefit of the treatment was evaluated using the |AUC1-6hour| values of the ipsilateral vehicle group (0% benefit) and all |AUC1-6hour| values of the contralateral sides of all three groups (100% benefit = maximal possible effect). Compared to these margins Labyrinthopeptin A1 achieved 95% benefit.

In conclusion, the compounds of the formula (I) significantly reduce tactile allodynia in the SNI mouse model of neuropathic pain.

## Claims

1. A compound of the formula (I) 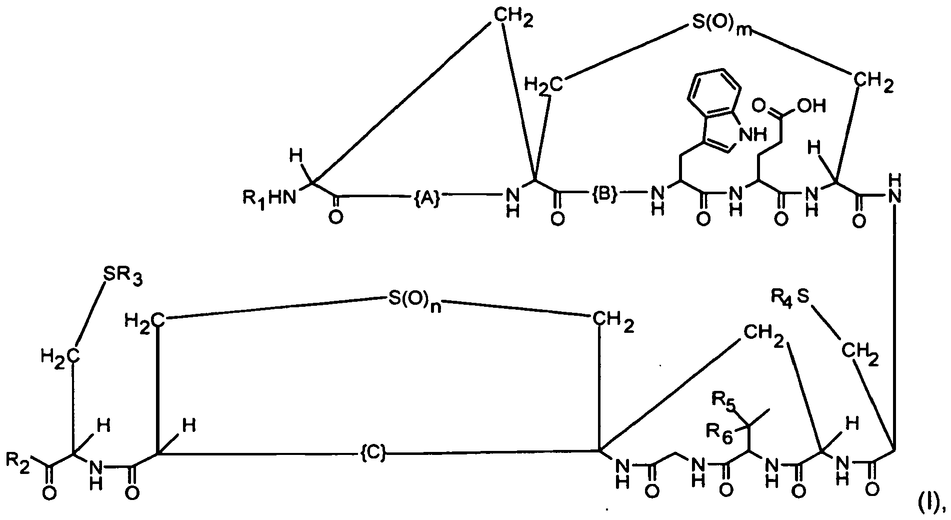 wherein
{A} is a group selected from 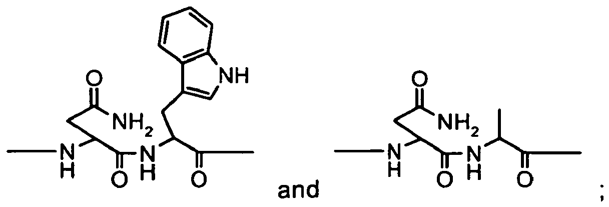
{B} is a group selected from 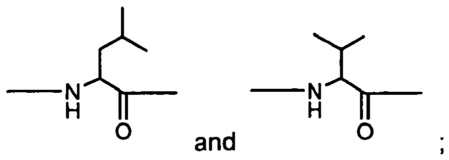
{C} is a group selected from 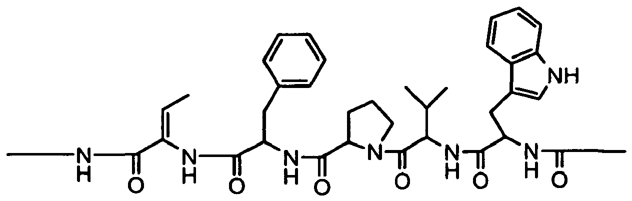 and 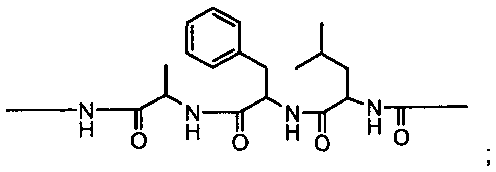
R₁ is a group R₁'or a group 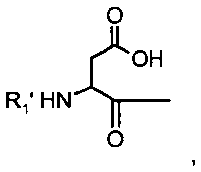 wherein R₁' is H, C(O)-(C₁-C₆)alkyl or C(O)-O-(C₁-C₆)alkyl;
R₂ is OH, NH₂, NH-(C₁-C₆)alkyl, NH-(C₁-C₄)alkylene-phenyl or NH-(C₁-C₄)alkylene-pyridyl;
R₃ and R₄ are independently of each other H, (C₁-C₆)alkyl, (C₁-C₆)alkylene-C(O)NH₂, (C₁-C₆)alkylene-C(O)NH(C₁-C₄)alkyl or (C₁-C₆)alkylene-C(O)N[(C₁-C₄)alkyl]₂, or R₃ and R₄ together with the S atoms to which they are attached form a disulfide group S-S;
R₅ and R₆ are independently of each other H or OH, or R₅ and R₆ together are =O;
m and n are independently of one another 0, 1 or 2;
with the proviso that if
{A} is 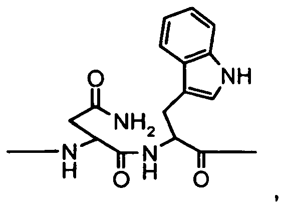
{B} is 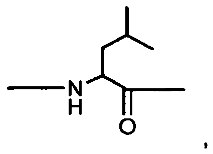 and
{C} is 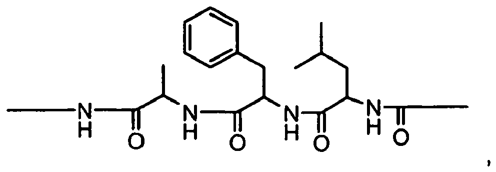
R₃ and R₄ may not form a disulfide group S-S together with the S atoms to which they are attached;
or a compound of the formula (IV) 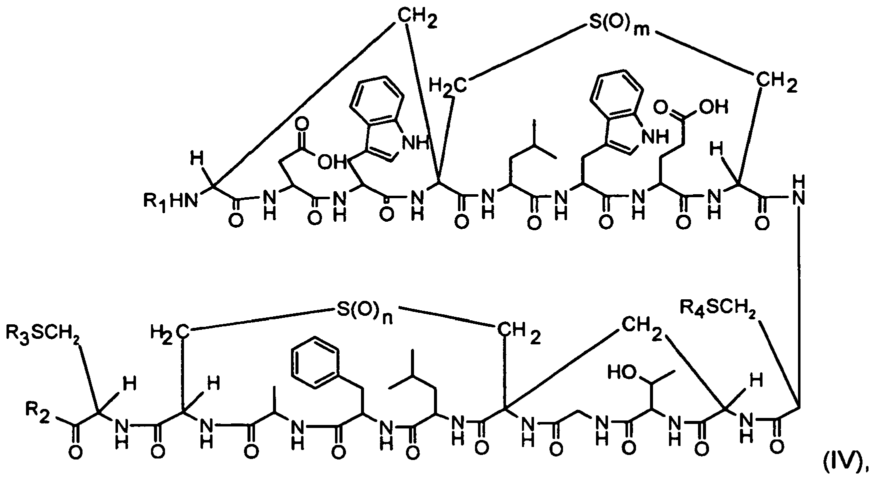 wherein
R₁ is H, C(O)-(C₁-C₆)alkyl or C(O)-O-(C₁-C₆)alkyl;
R₂ is OH, NH₂, NH-(C₁-C₆)-alkyl, N[(C₁-C₆)-alkyl]₂, NH-(C₁-C₄)-alkylene-phenyl or NH-(C₁-C₄)-alkylene-pyridyl;
R₃ and R₄ are independently from each other H, (CVC₆)alkyl or (C₁-C₄)-alkylene-C(O)NH₂;
m and n are independently of one another 0, 1 or 2;
in any stereochemical form, or a mixture of any stereochemical forms in any ratio, or a physiologically tolerable salt thereof.

2. A compound of the formula (I) according to claim 1, wherein
{A} is 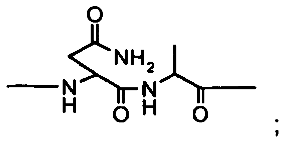
{B} is 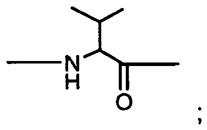
{C} is

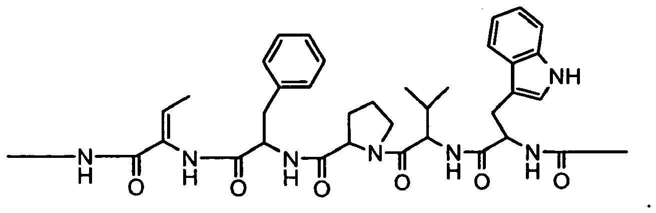

3. A compound of the formula (I) according to claim 1, wherein
{A} is 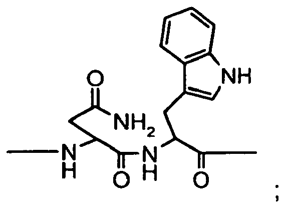
{B} is 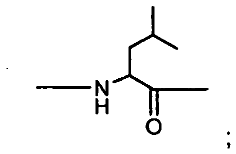
{C} is 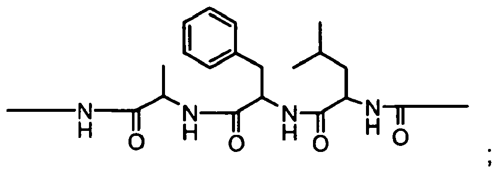 and
R₁ is preferably a group R₁'.

4. A compound of the formula (I) according to any one of claims 1 to 3, wherein R₁' is H.

5. A compound of the formula (I) or formula (IV) according to any one of claims 1 to 4, wherein R₂ is OH.

6. A compound of the formula (I) according to any one of claims 1 to 5, wherein R₃ and R₄ are independently of each other H, (C₁-C₆)alkyl, (C₁-C₆)alkylene-C(O)NH₂, or form a disulfide group S-S together with the S atoms to which they are attached.

7. A compound of the formula (I) according to any one of claims 1 to 6, wherein R₃ and R₄ are H or form a disulfide group S-S together with the S atoms to which they are attached.

8. A compound of the formula (I) according to any one of claims 1 to 7, wherein R₅ and R₆ are H or OH wherein if R₅ is OH then R₆ is H, and if R₅ is H then R₆ is OH, or R₅ and R₆ together are =O.

9. A compound of the formula (I) according to any one of claims 1 to 8, wherein R₅ is OH and R₆ is H, or R₅ is H and R₆ is OH.

10. A compound of the formula (I) according to any one of claims 1 to 9, **characterized by** the formula (II) 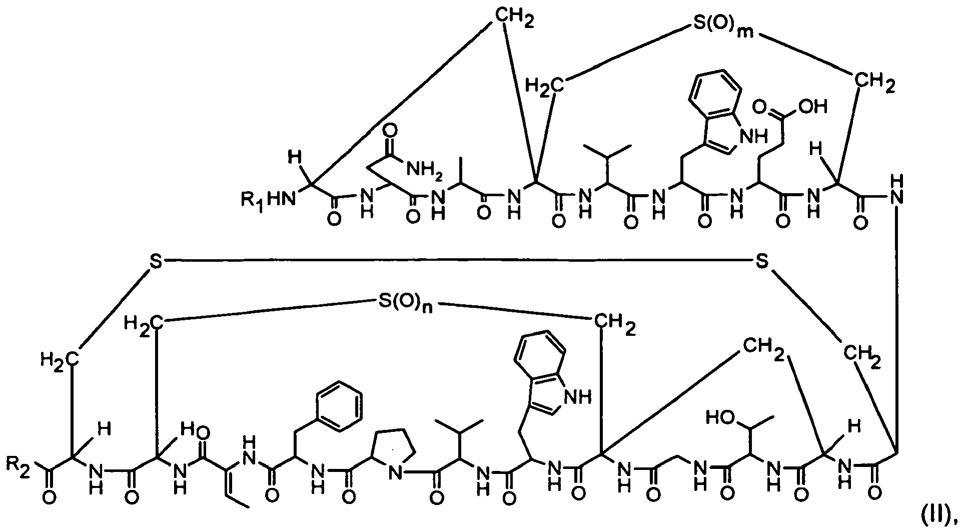 wherein R₁ is R₁' or a group 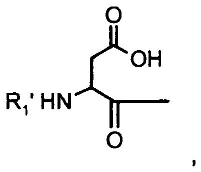 wherein R₁' is H, C(O)-(C₁-C₆)alkyl or C(O)-O-(C₁-C₆)alkyl, preferably H.

11. A compound of the formula (I) according to any one of claims 1 to 9, **characterized by** the formula (III) 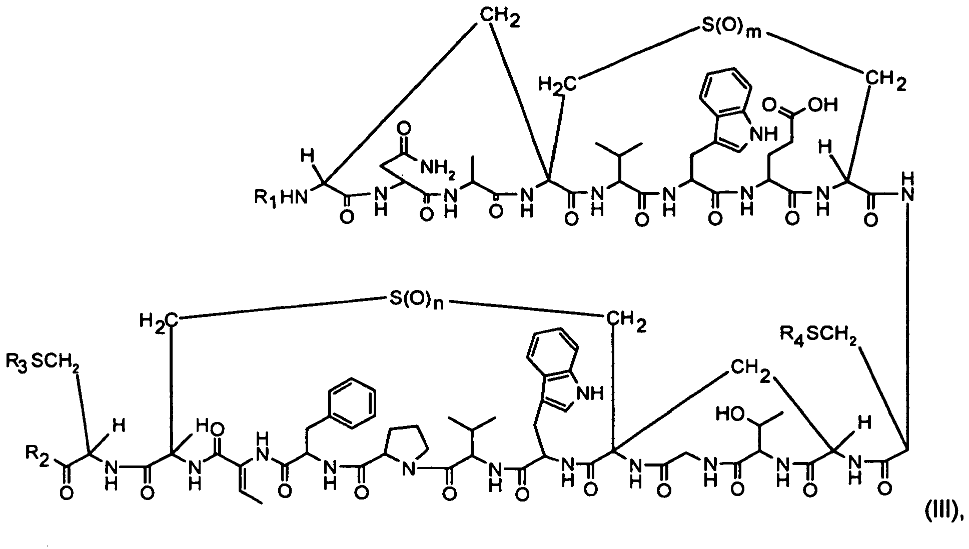 wherein
R₁ is R₁' or a group 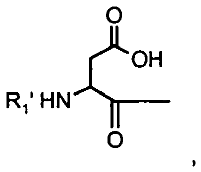 wherein R₁' is H, C(O)-(C₁-C₆)alkyl or C(O)-O-(C₁-C₆)alkyl, preferably H;
R₂ is OH, NH₂, NH-(C₁-C₆)-alkyl, N[(C₁-C₆)-alkyl]₂, NH-(C₁-C₄)-alkylene-phenyl or NH-(C₁-C₄)-alkylene-pyridyl, preferably R₂ is OH; and
R₃ and R₄ are independently from each other H, (C₁-C₆)alkyl or (C₁-C₄)-alkylene-C(O)NH₂.

12. A compound according to claim 1, **characterized by** the formula (IV) 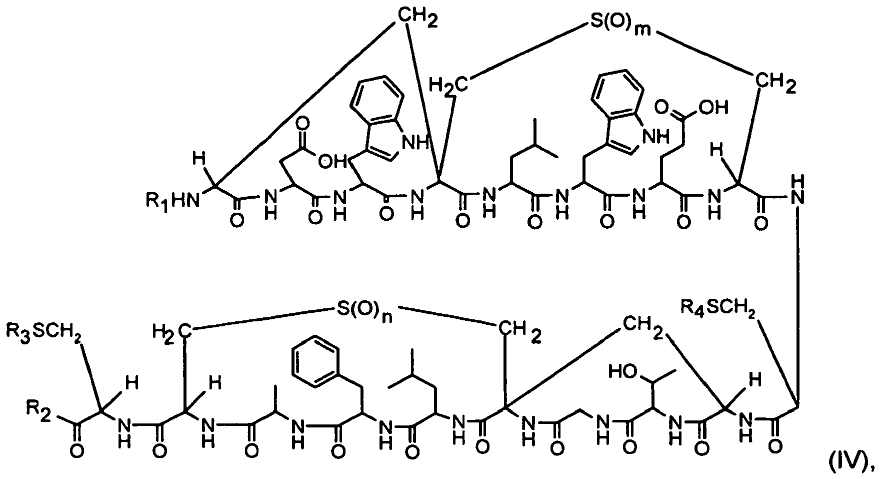 wherein
R₁ is H, C(O)-(C₁-C₆)alkyl or C(O)-O-(C₁-C₆)alkyl,
R₂ is OH, NH₂, NH-(C₁-C₆)-alkyl, N[(C₁-C₆)-alkyl]₂, NH-(C₁-C₄)-alkylene-phenyl or NH-(C₁-C₄)-alkylene-pyridyl, and
R₃ and R₄ are independently from each other H, (C₁-C₆)alkyl or (C₁-C₄)-alkylene-C(O)NH₂.

13. A compound of the formula (I) or formula (IV) according to any one of claims 1 to 12, wherein m and n are 0; or m and n are 2; or m is 0 and n is 2; or m is 2 and n is 0.

14. A compound of the formula (I) or formula (IV) according to any one of claims 1 to 13, wherein m and n are 0.

15. A process for preparing a compound of the formula (I) according to claim 1, comprising
a) fermenting the strain *Actinomadura namibiensis* (DSM 6313), or one of its variants and/or mutants, under suitable conditions in a culture medium until one or more of the compounds of the formula (I) accrue(s) in the culture medium,
b) isolating a compound of the formula (I) from the culture medium, and
c) derivatizing, where appropriate, the compound isolated in step b) and/or, where appropriate, converting the compound isolated in step b) or the derivative of the compound isolated in step b) into a physiologically tolerated salt.

16. The process according to claim 15, wherein the compound isolated in step b) is **characterized by** the formula (II) 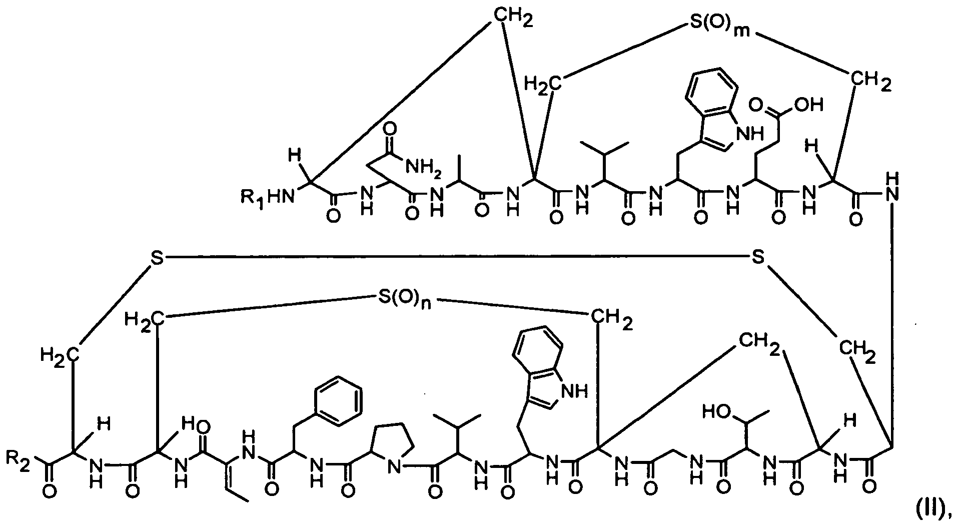 wherein
m and n are 0,
R₁ is R₁' or a group 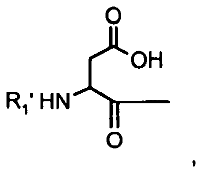 wherein R₁' is H, and
R₂ is OH.

17. A process for preparing a compound according to claim 1, wherein the compound is **characterized by** the formula (IV), 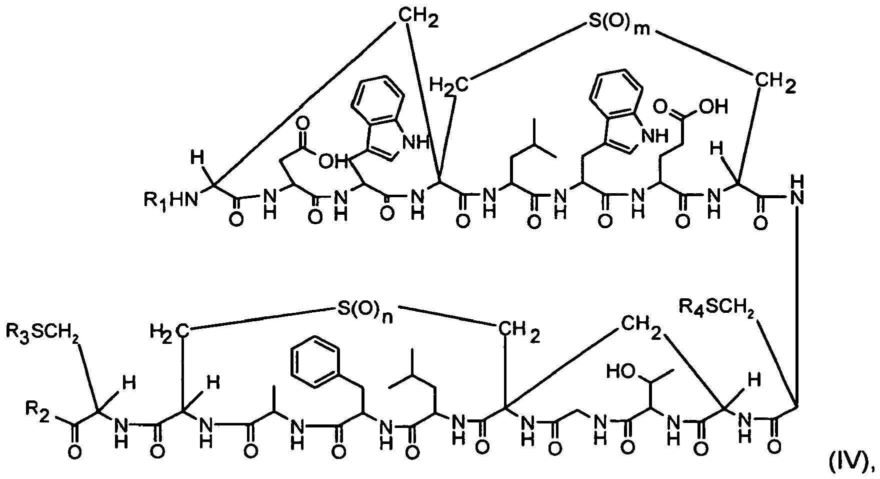 wherein
m and n are both 0,
R₁ is H,
R₂ is OH, and
R₃ and R₄ are independently of each other H, (C₁-C₆)alkyl, (C₁-C₆)alkylene-C(O)NH₂, (C₁-C₆)alkylene-C(O)NH(C₁-C₄)alkyl or (C₁-C₆)alkylene-C(O)N[(C₁-C₄)alkyl]₂, comprising
a) fermenting the strain *Actinomadura namibiensis* (DSM 6313), or one of its variants and/or mutants, under suitable conditions in a culture medium until the compound of formula (IV, i.e.) Labyrinthopeptin A2, accrues in the culture medium,
b) isolating the compound Labyrinthopeptin A2 from the culture medium, and
c) derivatizing the compound isolated in step b) and, where appropriate, converting the derivative of the compound isolated in step b) into a physiologically tolerated salt.

18. Use of a compound according to any one of claims 1 to 14 for the preparation of a medicament for the treatment of bacterial infections, viral infections and/or pain.

19. A pharmaceutical composition comprising at least one compound according to any one of claims 1 to 14 and at least one pharmaceutically acceptable carrier.

20. DNA coding for prepro-Labyrinthopeptin A2 having the nucleic acid sequence as shown in SEQ ID NO: 13.

21. Prepro-Labyrinthopeptin A2 having the amino acid sequence as shown in SEQ ID NO: 14.

22. Pro-Labyrinthopeptin A2 having the amino acid sequence as shown in SEQ. ID NO: 15.

23. DNA coding for prepro-Labyrinthopeptin A1 having the nucleic acid sequence as shown in SEQ. ID NO: 17.

24. Prepro-Labyrinthopeptin A1 having the amino acid sequence as shown in SEQ ID NO: 18.

25. Pro-Labyrinthopeptin A1 having the amino acid sequence as shown in SEQ. ID NO: 19.

## Patentansprüche

1. Verbindung der Formel (I) 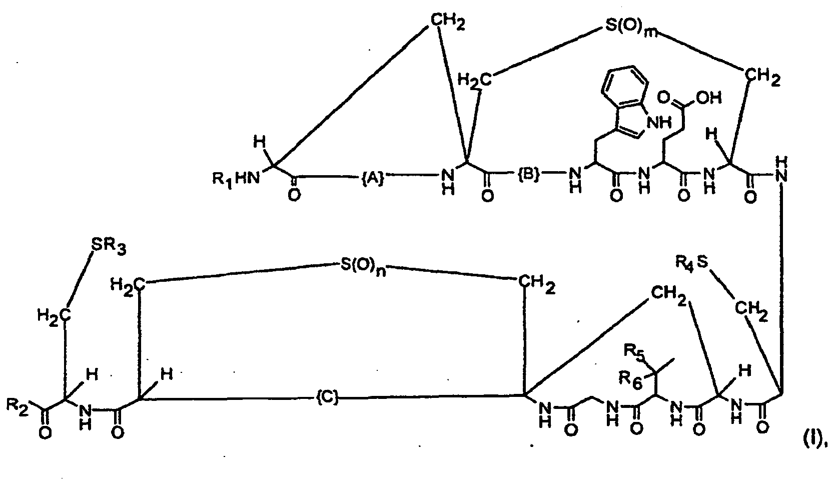 worin
{A} für eine unter 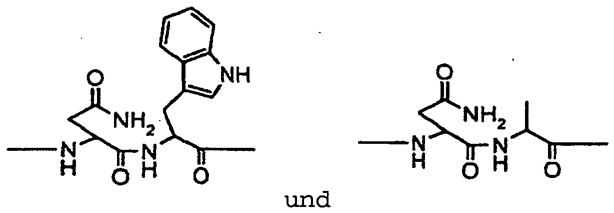 ausgewählte Gruppe steht;
{B} für eine unter 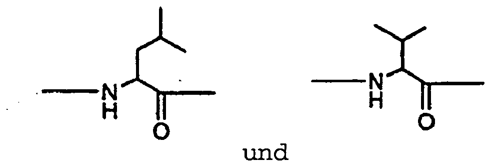 ausgewählte Gruppe steht;
{C} für eine unter 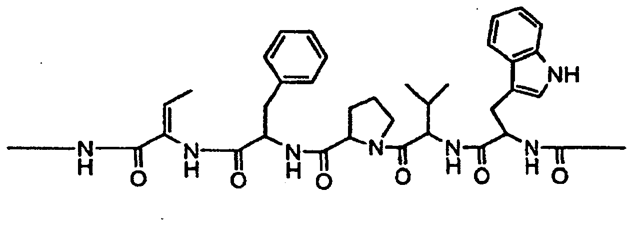 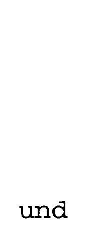 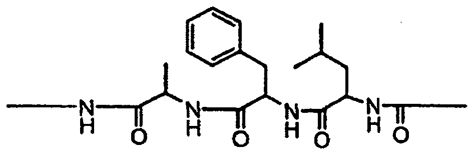 ausgewählte Gruppe steht;
R₁ für eine Gruppe R₁' oder eine Gruppe 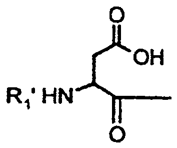 steht, worin R₁' für H, C(O)-(C₁-C₆)-Alkyl oder C(O)-O-(C₁-C₆)-Alkyl steht;
R₂ für OH, NH₂, NH-(C₁-C₆)-Alkyl, NH-(C₁-C₄) - Alkylenphenyl oder NH-(C₁-C₄)-Alkylenpyridyl steht;
R₃ und R₄ unabhängig voneinander für H, (C₁-C₆)-Alkyl, (C₁-C₆) -Alkylen-C (O)NH₂, (C₁-C₆) -Alkylen-C(O)NH(C₁-C₄)-alkyl oder (C₁-C₆)-Alkylen-C(O)N[(C₁-C₄)-alkyl]₂ stehen oder R₃ und R₄ zusammen mit den S-Atomen, an die sie gebunden sind, eine Disulfidgruppe S-S bilden;
R₅ und R₆ unabhängig voneinander für H oder OH stehen oder R₅ und R₆ zusammen für =O stehen;
m und n unabhängig voneinander für 0, 1 oder 2 stehen;
mit der Maßgabe, dass dann, wenn
{A} für 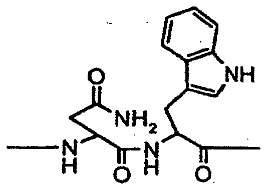 steht,
{B} für 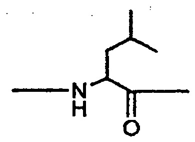 steht und
{C} für 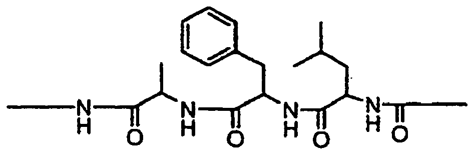 steht,
R₃ und R₄ nicht zusammen mit den S-Atomen, an die sie gebunden sind, eine Disulfidgruppe S-S bilden;
oder eine Verbindung der Formel (IV) 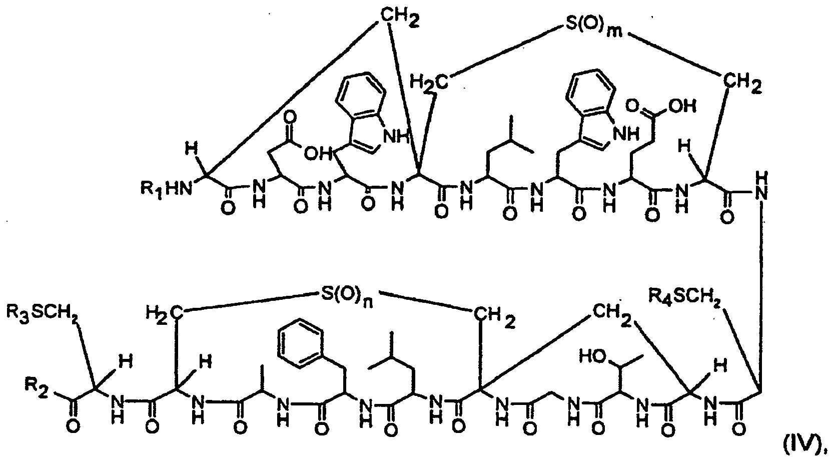 worin
R₁ für H, C(O)-(C₁-C₆)-Alkyl oder C(O)-O-(C₁-C₆)-Alkyl steht;
R₂ für OH, NH₂, NH-(C₁-C₆) -Alkyl, N[(C₁-C₆)-Alkyl]₂, NH-(C₁-C₄)-Alkylenphenyl oder NH-(C₁-C₄)-Alkylenpyridyl steht;
R₃ und R₄ unabhängig voneinander für H, (C₁-C₆) - Alkyl oder (C₁-C₄)-Alkylen-C(O)NH₂ stehen;
m und n unabhängig voneinander für 0, 1 oder 2 stehen;
in beliebiger stereochemischer Form oder ein Gemisch beliebiger stereochemischer Formen in einem beliebigen Verhältnis oder ein physiologisch tolerierbares Salz davon.

2. Verbindung der Formel (I) nach Anspruch 1, worin
{A} für 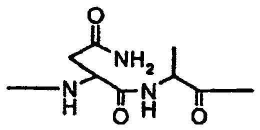 steht;
{B} für 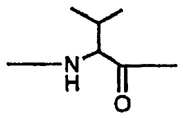 steht;
{C} für 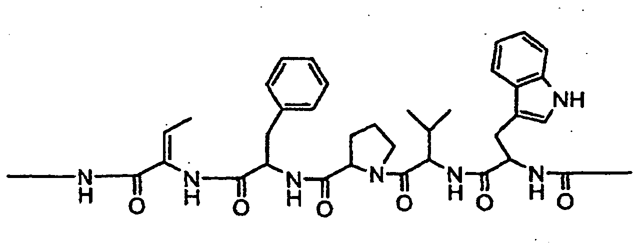 steht.

3. Verbindung der Formel (I) nach Anspruch 1, worin
{A} für 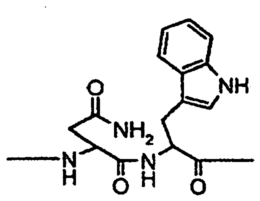 steht;
{B} für 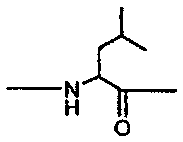 steht;
{C} für 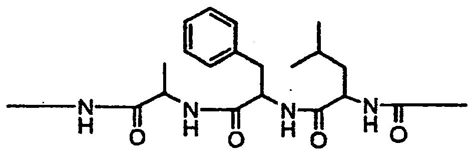 steht und
R₁ vorzugsweise für eine Gruppe R₁' steht.

4. Verbindung der Formel (I) nach einem der Ansprüche 1 bis 3, worin R₁' für H steht.

5. Verbindung der Formel (I) oder (IV) nach einem der Ansprüche 1 bis 4, worin R₂ für OH steht.

6. Verbindung der Formel (I) nach einem der Ansprüche 1 bis 5, worin R₃ und R₄ unabhängig voneinander für H, (C₁-C₆)-Alkyl oder (C₁-C₆)-Alkylen-C(O)NH₂ stehen oder zusammen mit den S-Atomen, an die sie gebunden sind, eine Disulfidgruppe S-S bilden.

7. Verbindung der Formel (I) nach einem der Ansprüche 1 bis 6, worin R₃ und R₄ für H stehen oder zusammen mit den S-Atomen, an die sie gebunden sind, eine Disulfidgruppe S-S bilden.

8. Verbindung der Formel (I) nach einem der Ansprüche 1 bis 7, worin R₅ und R₆ für H oder OH stehen, wobei dann, wenn R₅ für OH steht, R₆ für H steht und dann, wenn R₅ für H steht, R₆ für OH steht, oder R₅ und R₆ zusammen für =O stehen.

9. Verbindung der Formel (I) nach einem der Ansprüche 1 bis 8, worin R₅ für OH steht und R₆ für H steht oder R₅ für H steht und R₆ für OH steht.

10. Verbindung der Formel (I) nach einem der Ansprüche 1 bis 9, **gekennzeichnet durch** die Formel (II) 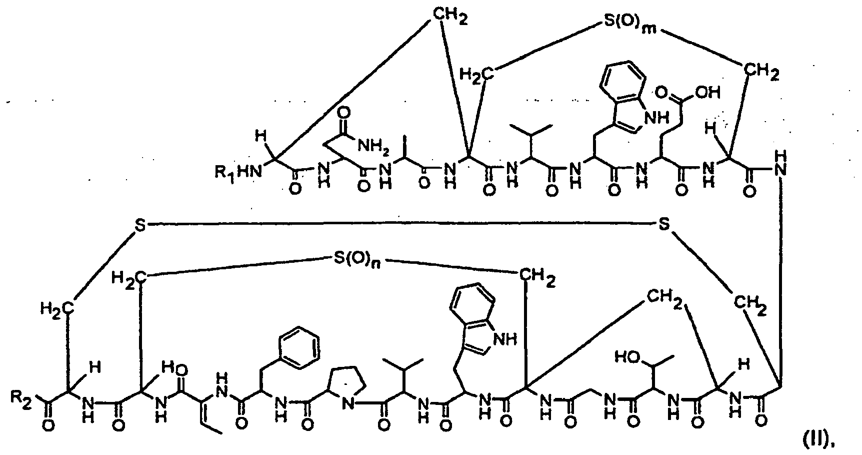 worin R₁ für R₁' oder eine Gruppe 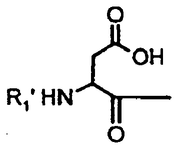 steht, worin R₁' für H, C(O)-(C₁-C₆)-Alkyl oder C(O)-O-(C₁-C₆)-Alkyl, vorzugsweise H, steht.

11. Verbindung der Formel (I) nach einem der Ansprüche 1 bis 9, **gekennzeichnet durch** die Formel (III) 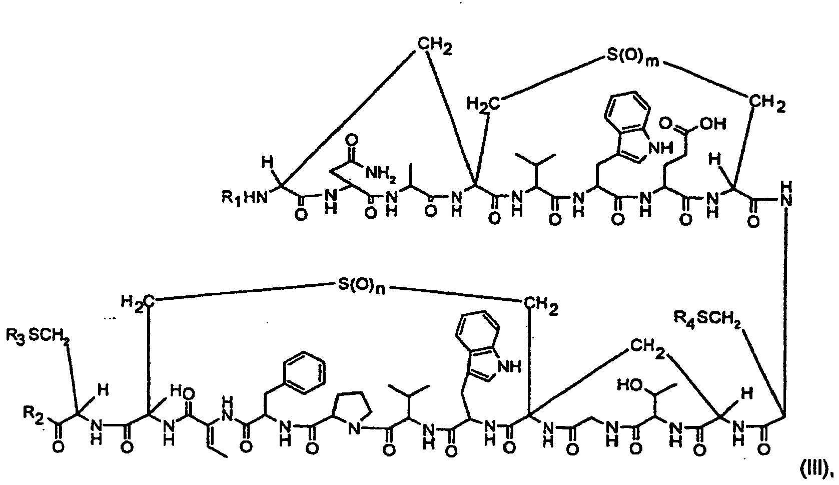 worin R₁ für R₁' oder eine Gruppe 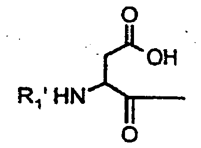 steht, worin R₁' für H, C(O)-(C₁-C₆)-Alkyl oder C(O)-O-(C₁-C₆)-Alkyl, vorzugsweise H, steht;
R₂ für OH, NH₂, NH-(C₁-C₆) -Alkyl, N[(C₁-C₆)-Alkyl]₂, NH-(C₁-C₄)-Alkylenphenyl oder NH- (C₁-C₄)-Alkylenpyridyl steht und R₂ vorzugsweise für OH steht und
R₃ und R₄ unabhängig voneinander für H, (C₁-C₆)-Alkyl oder (C₁-C₄)-Alkylen-C(O)NH₂ stehen.

12. Verbindung nach Anspruch 1, **gekennzeichnet durch** die Formel (IV) 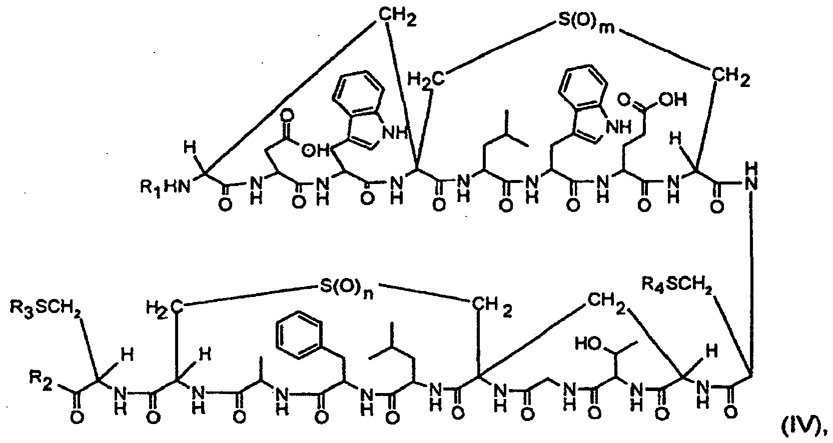 worin
R₁ für H, C(O)-(C₁-C₆)-Alkyl oder C(O)-O-(C₁-C₆)-Alkyl steht;
R₂ für OH, NH₂, NH-(C₁-C₆)-Alkyl, N[(C₁-C₆)-Alkyl]₂, NH- (C₁-C₄)-Alkylenphenyl oder NH- (C₁-C₄)-Alkylenpyridyl steht und
R₃ und R₄ unabhängig voneinander für H, (C₁-C₆)-Alkyl oder (C₁-C₄)-Alkylen-C(O)NH₂ stehen.

13. Verbindung der Formel (I) oder Formel (IV) nach einem der Ansprüche 1 bis 13, worin m und n für 0 stehen oder m und n für 2 stehen oder m für 0 steht und n für 2 steht oder m für 2 steht und n für 0 steht.

14. Verbindung der Formel (I) oder Formel (IV) nach einem der Ansprüche 1 bis 13, worin m und n für 0 stehen.

15. Verfahren zur Herstellung einer Verbindung der Formel (I) nach Anspruch 1, bei dem man
a) den Stamm *Actinomadura namibiensis* (DSM 6313) oder eine seiner Varianten und/oder Mutanten unter geeigneten Bedingungen in einem Kulturmedium fermentiert, bis sich eine oder mehrere der Verbindungen der Formel (I) im Kulturmedium ansammelt bzw. ansammeln,
b) eine Verbindung der Formel (I) aus dem Kulturmedium isoliert und
c) die in Schritt b) isolierte Verbindung gegebenenfalls derivatisiert und/oder gegebenenfalls die in Schritt b) isolierte Verbindung oder das Derivat der in Schritt b) isolierten Verbindung in ein physiologisch toleriertes Salz umwandelt.

16. Verfahren nach Anspruch 15, bei dem die in Schritt b) isolierte Verbindung durch die Formel (II) 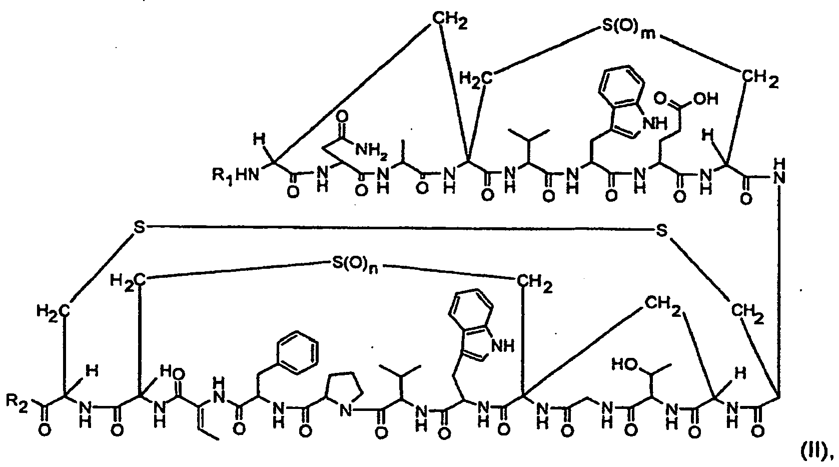 worin
m und n für 0 stehen,
R₁ für R₁' oder eine Gruppe 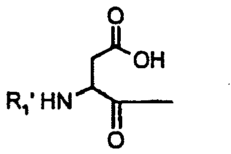 steht, worin R₁' für H steht, und
R₂ für OH steht,
gekennzeichnet ist.

17. Verfahren zur Herstelung einer Verbindung nach Anspruch 1, bei dem die Verbindung durch die Formel (IV) 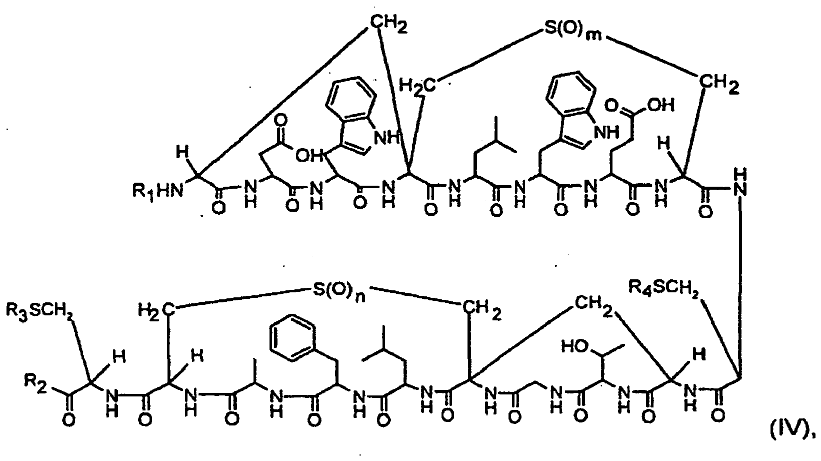 worin
m und n beide für 0 stehen,
R₁ für H steht,
R₂ für OH steht und
R₃ und R₄ unabhängig voneinander für H, (C₁-C₆)-Alkyl, (C₁-C₆) -Alkylen-C(O)NH₂, (C₁-C₆) -Alkylen-C(O)NH(C₁-C₄)-alkyl oder (C₁-C₆)-Alkylen-C(O)N[(C₁-C₄)-alkyl]₂ stehen,
gekennzeichnet ist,
bei dem man
a) den Stamm *Actinomadura namibiensis* (DSM 6313) oder eine seiner Varianten und/oder Mutanten unter geeigneten Bedingungen in einem Kulturmedium fermentiert, bis sich die Verbindung der Formel (IV), d.h. Labyrinthopeptin A2, im Kulturmedium ansammelt,
b) die Verbindung Labyrinthopeptin A2 aus dem Kulturmedium isoliert und
c) die in Schritt b) isolierte Verbindung gegebenenfalls derivatisiert und/oder gegebenenfalls das Derivat der in Schritt b) isolierten Verbindung in ein physiologisch toleriertes Salz umwandelt.

18. Verwendung einer Verbindung nach einem der Ansprüche 1 bis 14 zur Herstellung eines Arzneimittels für die Behandlung von bakteriellen Infektionen, Virusinfektionen und/oder Schmerzen.

19. Pharmazeutische Zusammensetzung, umfassend mindestens eine Verbindung nach einem der Ansprüche 1 bis 14 und mindestens einen pharmazeutisch unbedenklichen Träger.

20. Für Präpro-Labyrinthopeptin A2 codierende DNA mit der Nucleinsäuresequenz gemäß SEQ ID NO: 13.

21. Präpro-Labyrinthopeptin A2 mit der Aminosäuresequenz gemäß SEQ ID NO: 14.

22. Pro-Labyrinthopeptin A1 mit der Aminosäuresequenz gemäß SEQ ID NO: 15.

23. Für Präpro-Labyrinthopeptin A1 codierende DNA mit der Nucleinsäuresequenz gemäß SEQ ID NO: 17.

24. Präpro-Labyrinthopeptin A1 mit der Aminosäuresequenz gemäß SEQ ID NO: 18.

25. Pro-Labyrinthopeptin A1 mit der Aminosäuresequenz gemäß SEQ ID NO: 19.

## Revendications

1. Composé de formule (I) 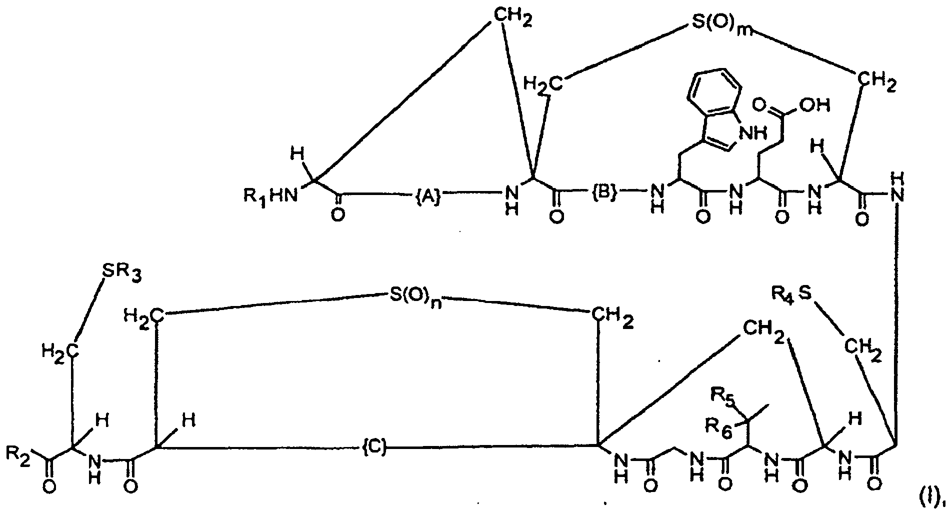 où
{A} représente un groupement choisi parmi 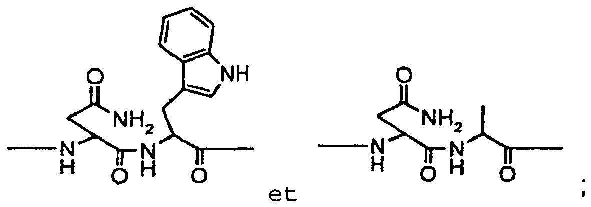
{B} représente un groupement choisi parmi 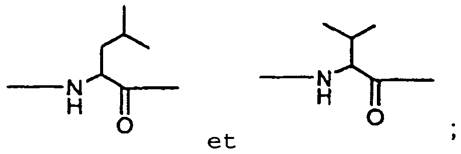
{C} représente un groupement choisi parmi 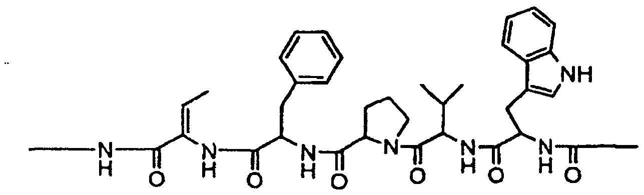 et 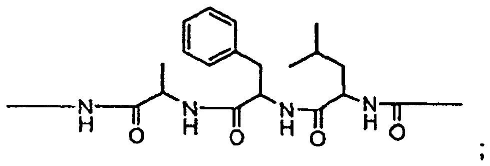
R₁ représente un groupement R₁' ou un groupement 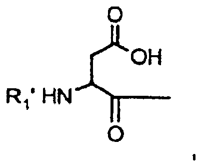 où R₁' représente H ou un groupement C(O)-alkyle en C₁-C₆ ou C(O)-O-alkyle en C₁-C₆ ;
R₂ représente un groupement OH, NH₂, NH-alkyle en C₁-C₆, NH-(alkylène en C₁-C₄)-phényle ou NH-(alkylène en C₁-C₄) - pyridyle ;
chacun des radicaux R₃ et R₄ représente indépendamment de l'autre H ou un groupement alkyle en C₁-C₆, (alkylène en C₁-C₆)-C(O)NH₂, (alkylène en C₁-C₆)-C(O)NH-alkyle en C₁-C₄ ou (alkylène en C₁-C₆)-C(O)N [alkyle en C₁-C₄]₂, ou R₃ et R₄ représentent ensemble et avec les atomes S auxquels ils sont liés un groupement disulfure S-S ;
chacun des radicaux R₅ et R₆ représente indépendamment de l'autre H ou OH, ou R₅ et R₆ forment ensemble =O ;
chacun des nombres m et n est égal indépendamment de l'autre à 0, 1 ou 2 ;
à la condition que lorsque
{A} représente 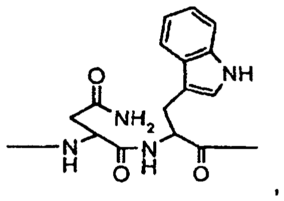
{B} représente 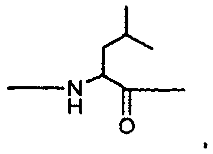 et
{C} représente 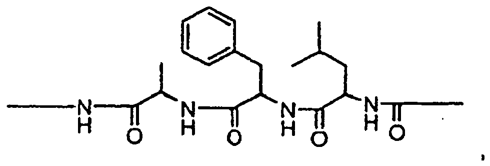
R₃ et R₄ ne peuvent pas former de groupement disulfure S-S ensemble et avec les atomes S auxquels ils sont liés ;
ou un composé de formule (IV) 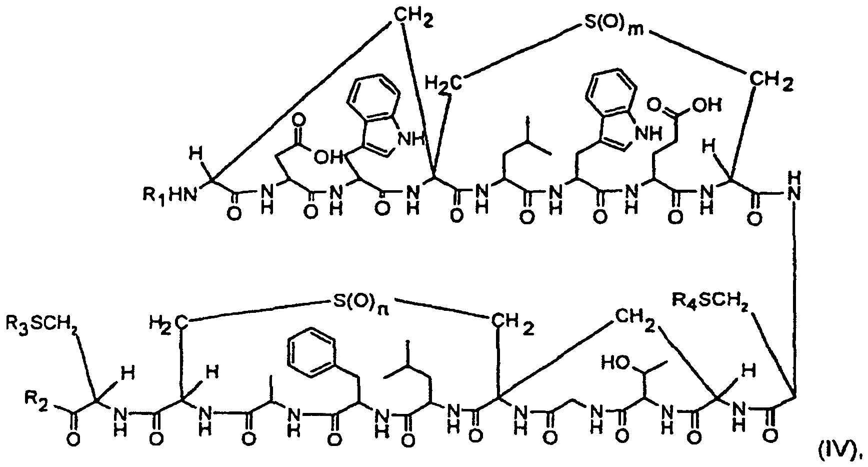 où
R₁ représente H ou un groupement C(O)-alkyle en C₁-C₆ ou C(O)-O-alkyle en C₁-C₆ ;
R₂ représente un groupement OH, NH₂, NH-alkyle en C₁-C₆, N[alkyle en C₁-C₆]₂, NH-(alkylène en C₁-C₄)-phényle ou NH-(alkylène en C₁-C₄)-pyridyle ;
chacun des radicaux R₃ et R₄ représente indépendamment de l'autre H ou un groupement alkyle en C₁-C₆ ou (alkylène en C₁-C₄)-C(O)NH₂ ;
chacun des nombres m et n est égal indépendamment de l'autre à 0, 1 ou 2 ;
sous toute forme stéréochimique, ou sous forme d'un mélange de formes stéréochimiques dans tout rapport, ou l'un de leurs sels de qualité physiologique.

2. Composé de formule (I) conforme à la revendication 1, où
{A} représente 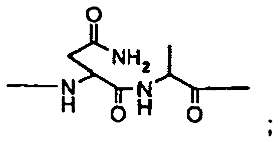
{B} représente 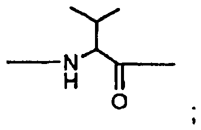
{C} représente

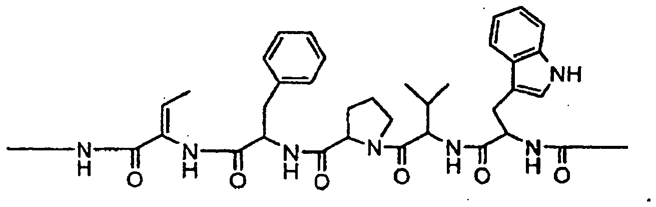

3. Composé de formule (I) conforme à la revendication 1, où
{A} représente 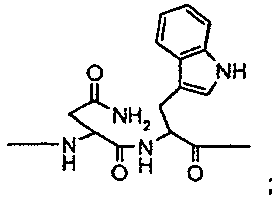
{B} représente 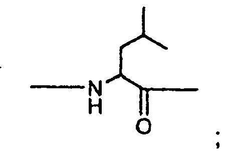
{C} représente 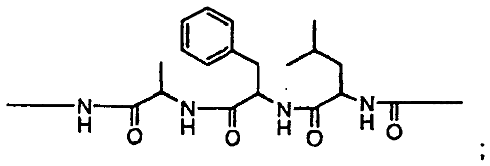 et
R₁ représente préférentiellement un groupement R₁',

4. Composé de formule (I) conforme à l'une quelconque des revendications 1 à 3, où R₁' représente H.

5. Composé de formule (I) ou de formule (IV) conforme à l'une quelconque des revendications 1 à 4, où R₂ représente OH.

6. Composé de formule (I) conforme à l'une quelconque des revendications 1 à 5, où chacun des radicaux R₃ et R₄ représente indépendamment de l'autre H ou un groupement alkyle en C₁-C₆, (alkylène en C₁-C₆) - C(O)NH₂, ou les deux radicaux forment ensemble et avec les atomes S auxquels ils sont liés un groupement disulfure S-S.

7. Composé de formule (I) conforme à l'une quelconque des revendications 1 à 6, où chacun des radicaux R₃ et R₄ représente H ou les deux radicaux forment ensemble et avec les atomes S auxquels ils sont liés un groupement disulfure S-S.

8. Composé de formule (I) conforme à l'une quelconque des revendications 1 à 7, où chacun des radicaux R₅ et R₆ représente H ou OH, à la condition que lorsque R₅ représente OH, alors R₆ représente H, et lorsque R₅ représente H, alors R₆ représente OH, ou R₅ et R₆ forment ensemble =O.

9. Composé de formule (I) conforme à l'une quelconque des revendications 1 à 8, où R₅ représente OH et R₆ représente H, ou R₅ représente H et R₆ représente OH.

10. Composé de formule (I) conforme à l'une quelconque des revendications 1 à 9, **caractérisé par** la formule (II) 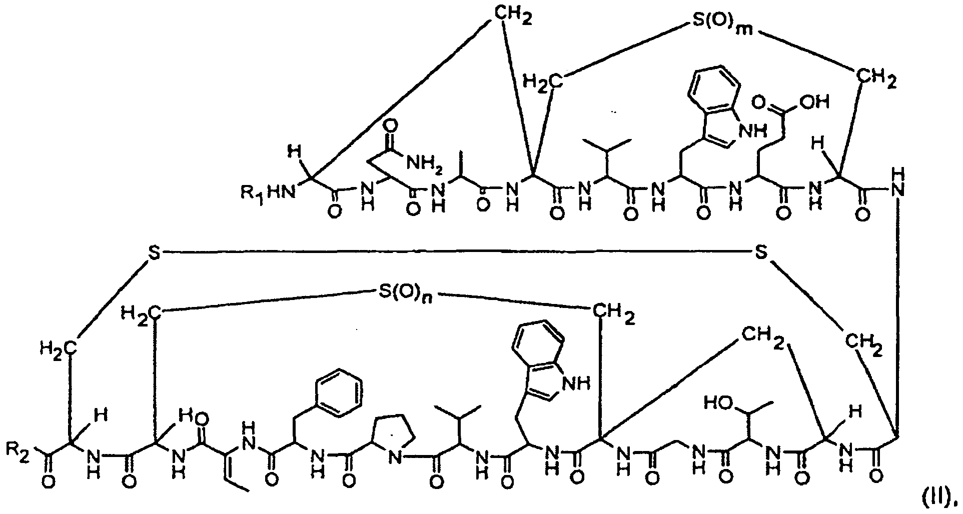 où R₁ représente R₁' ou un groupement 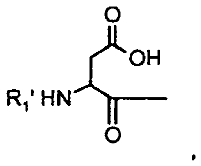 où R₁' représente H ou un groupement C(O)-alkyle en C₁-C₆ ou C(O)-O-alkyle en C₁-C₆, préférentiellement H.

11. Composé de formule (I) conforme à l'une quelconque des revendications 1 à 9, **caractérisé par** la formule (III) 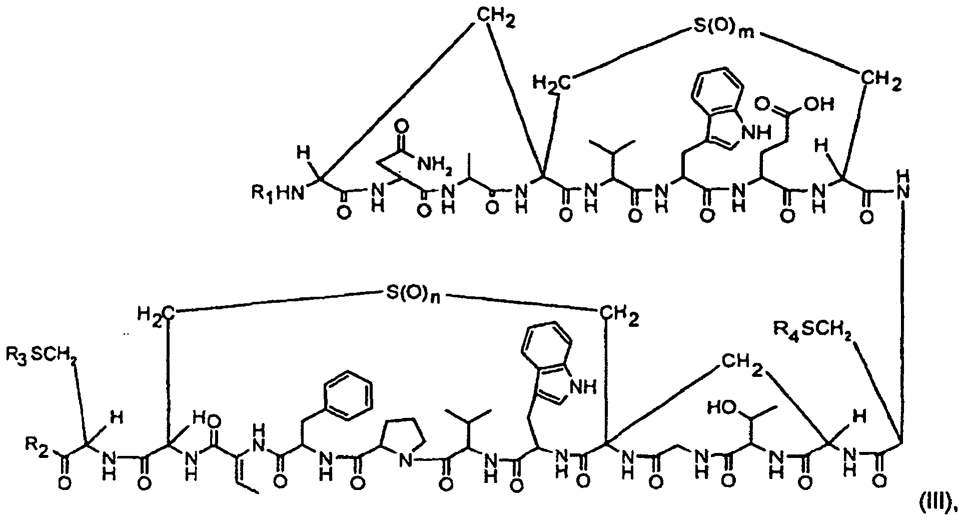 où
R₁ représente R₁' ou un groupement 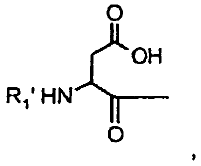 où R₁' représente H ou un groupement C(O)-alkyle en C₁-C₆ ou C(O)-O-alkyle en C₁-C₆, préférentiellement H ;
R₂ représente un groupement OH, NH₂, NH-alkyle en C₁-C₆, N[alkyle en C₁-C₆]₂, NH-(alkylène en C₁-C₄)-phényle ou NH-(alkylène en C₁-C₄)-pyridyle, préférentiellement R₂ représente OH ; et
chacun des radicaux R₃ et R₄ représente indépendamment de l'autre H ou un groupement alkyle en C₁-C₆ ou (alkylène en C₁-C₄)-C(O)NH₂.

12. Composé conforme à la revendication 1, **caractérisé par** la formule (IV) 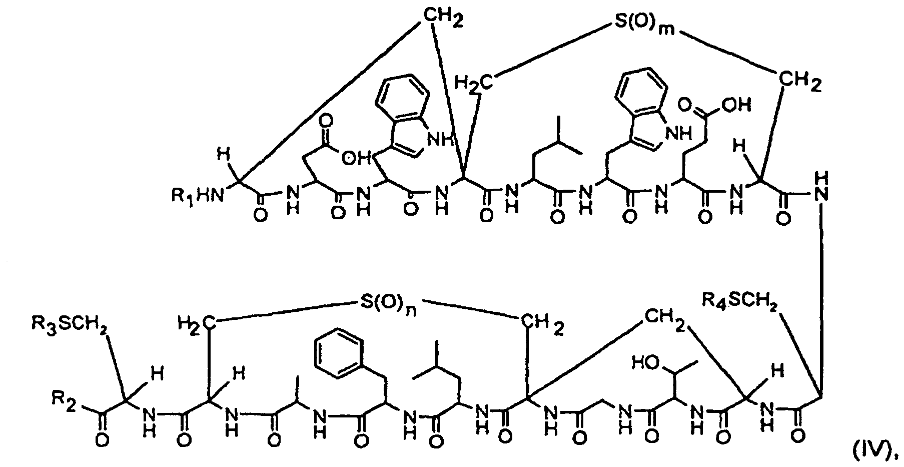 où
R₁ représente H ou un groupement C(O)-alkyle en C₁-C₆ ou C(O)-O-alkyle en C₁-C₆,
R₂ représente un groupement OH, NH₂, NH-alkyle en C₁-C₆, N[alkyle en C₁-C₆]₂, NH-(alkylène en C₁-C₄)-phényle ou NH-(alkylène en C₁-C₄)-pyridyle, et
chacun des radicaux R₃ et R₄ représente indépendamment de l'autre H ou un groupement alkyle en C₁-C₆ ou (alkylène en C₁-C₄)-C(O)NH₂.

13. Composé de formule (I) ou de formule (IV) conforme à l'une quelconque des revendications 1 à 12, où chacun des nombres m et n est égal à 0 ; chacun des nombres m et n est égal à 2 ; ou m est égal à 0 et n est égal à 2 ; ou m est égal à 2 et n est égal à 0.

14. Composé de formule (I) ou de formule (IV) conforme à l'une quelconque des revendications 1 à 13, où chacun des nombres m et n est égal à 0.

15. Procédé de synthèse d'un composé de formule (I) conforme à la revendication 1, comprenant
a) la fermentation de la souche *Actinomadura namibiensis* (DSM 6313), ou de l'une de ses variantes et/ou mutants, dans des conditions adaptées au sein d'un milieu de culture, jusqu'à ce que la quantité d'un ou de plusieurs des composés de formule (I) s'accroisse dans le milieu de culture,
b) l'isolement d'un composé de formule (I) d'avec le milieu de culture, et
c) la dérivation, le cas échéant, du composé isolé dans l'étape b), et/ou, le cas échéant, la conversion du composé isolé dans l'étape b) ou du dérivé du composé isolé dans l'étape b) dans un sel de qualité physiologique.

16. Procédé conforme à la revendication 15, où le composé isolé dans l'étape b) est **caractérisé par** la formule (II) 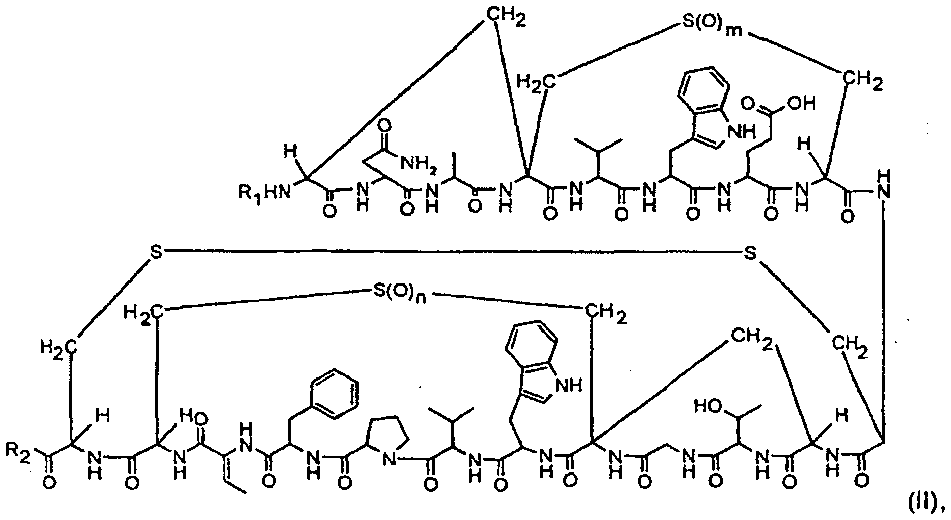 où chacun des nombres m et n est égal à 0, R₁ représente R₁' ou un groupement 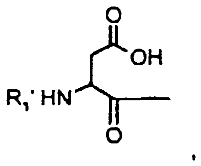 où R₁' représente H, et R₂ représente OH.

17. Procédé de synthèse d'un composé conforme à la revendication 1, où le composé est **caractérisé par** la formule (IV), 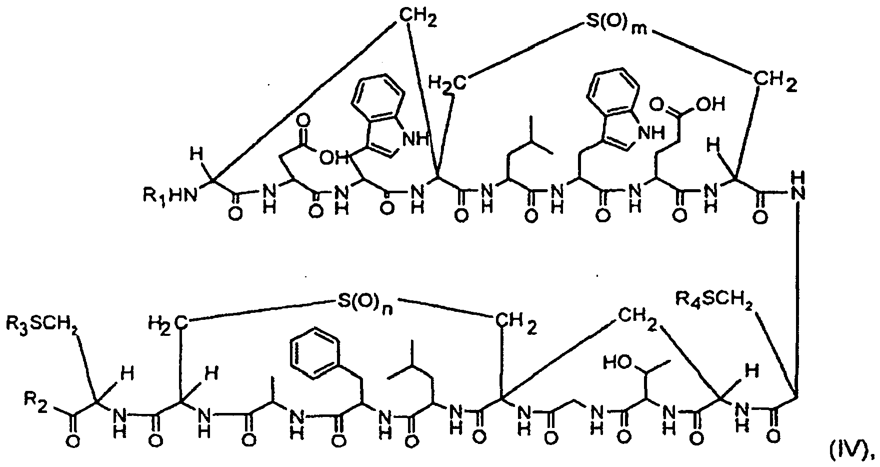 où
m et n sont tous deux égaux à 0,
R₁ représente H,
R₂ représente OH, et
chacun des radicaux R₃ et R₄ représente indépendamment de l'autre H ou un groupement alkyle en C₁-C₆, (alkylène en C₁-C₆)-C(O)NH₂, (alkylène en C₁-C₆)-C(O)NH-alkyle en C₁-C₄ ou (alkylène en C₁-C₆)-C(O)N[alkyle en C₁-C₄]₂,
comprenant
a) la fermentation de la souche *Actinomadura namibiensis* (DSM 6313), ou l'une de ses variantes et/ou mutants, dans des conditions adaptées au sein d'un milieu de culture, jusqu'à ce que la quantité du composé de formule (IV), c'est-à-dire la labyrinthopeptine A2, s'accroisse dans le milieu de culture,
b) l'isolement de la labyrinthopeptine A2 d'avec le milieu de culture, et
c) la dérivation du composé isolé dans l'étape b) et, le cas échéant, la conversion du dérivé du composé isolé dans l'étape b) en un sel de qualité physiologique.

18. Emploi d'un composé conforme à l'une quelconque des revendications 1 à 14 dans l'élaboration d'un médicament destiné au traitement d'infections bactériennes, d'infections virales et/ou de la douleur.

19. Composition pharmaceutique comprenant au moins un composé conforme à l'une quelconque des revendications 1 à 14 ainsi qu'au moins un vecteur de qualité pharmaceutique.

20. ADN codant pour la prépro-labyrinthopeptine A2 comportant la séquence d'acides nucléiques de SEQ ID NO: 13.

21. Prépro-labyrinthopeptine A2 comportant la séquence d'acides aminés de SEQ ID NO: 14.

22. Pro-labyrinthopeptine A2 comportant la séquence d'acides aminés de SEQ ID NO: 15.

23. ADN codant pour la prépro-labyrinthopeptine A1 comportant la séquence d'acides nucléiques de SEQ ID NO: 17.

24. Prépro-labyrinthopeptine A1 comportant la séquence d'acides aminés de SEQ ID NO: 18.

25. Prépro-labyrinthopeptine A1 comportant la séquence d'acides aminés de SEQ ID NO: 19.


### SEQUENCE LISTING

<110> sanofi-Aventis
<120> Highly bridged peptides from Actinomadura namibiensis
<130> DE2008/027
<160> 19
<170> PatentIn version 3.3
<210> 1
   <211> 20
   <212> PRT
   <213> Artificial
<220>
   <223> ser1 bridged with Ser4 via ch2
   Ser4 bridged with ser1 via ch2
   Ser4 bridged with Cys8 via S(O)m wherein m is 0, 1 or 2 Ser4 bridged with Ser4 via S(O)m wherein m is 0, 1 or 2 Cys9 optionally bridged with Cys20 via S-S
   Ser10 bridged with ser13 via CH2
   Ser13 bridged with Ser10 via CH2
   Ser13 bridged with Cys19 via S(O)n wherein n is 0, 1 or 2
   Cys19 bridged with Ser13 via S(O)n wherein n is 0, 1 or 2
   Cys20 optionally bridged with Cys9 via S-S
<400> 1
   Ser Asn Ala Ser Val Trp Glu Cys Cys Ser Thr Gly Ser Trp Val Pro
   1               5                   10                  15
   Phe Thr Cys Cys
               20
<210> 2
   <211> 18
   <212> PRT
   <213> Artificial
<220>
   <223> Ala1 bridged with Ala4 via CH2
   Ala4 bridged with Ala1 via CH2
   Ala4 bridged with Ala8 via S(O)m wherein m is 0, 1 or 2 Ala8 bridged with Ala4 via S(O)m wherein m is 0, 1 or 2 Cys9 bridged with Cys18 via S-S
   Ala10 bridged with Ala13 via CH2
   Ala13 bridged with Ala10 via CH2
   ala13 bridged with Ala17 via S(O)n wherein n is 0, 1 or 2
   Ala19 bridged with Ala13 via S(O)n wherein n is 0, 1 or 2
   Cys18 optionally bridged with Cys9 via S-S
<400> 2
   Ala Asp Trp Ala Leu Trp Glu Ala Cys Ala Thr Gly Ala Leu Phe Ala
   1               5                   10                  15
   Ala Cys
<210> 3
   <211> 18
   <212> PRT
   <213> Artificial
<220>
   <223> Ser1 bridged with ser4 via CH2
   Ser4 bridged with ser1 via CH2
   Ser4 bridged with Cys8 via S(O)m wherein m is 0, 1 or 2
   Cys8 bridged with Ser4 via S(O)m wherein m is 0, 1 or 2 Ser10 bridged with Ser13 via CH2
   Ser13 bridged with Ser10 via CH2
   Ser13 bridged with Ser17 via S(O)n wherein n is 0, 1 or 2
   Ser19 bridged with Ser13 via S(O)n wherein n is 0, 1 or 2
<400> 3
   Ser Asp Trp Ser Leu Trp Glu Cys Cys Ser Thr Gly Ser Leu Phe Ala
   1               5                   10                  15
   Cys Cys
<210> 4
   <211> 34
   <212> DNA
   <213> Artificial
<220>
   <223> Forward Primer for Labyrinthopeptin A2
<220>
   <221> misc_feature
   <222> (27)..(27)
   <223> n is a, c, g, or t
<220>
   <221> misc_feature
   <222> (30)..(30)
   <223> n is a, c, g, or t
<400> 4
   caggaaacag ctatgaccga ytggwsnytn tggg 34
<210> 5
   <211> 35
   <212> DNA
   <213> Artificial
<220>
   <223> Reversed Primer for Labyrinthopeptin A2
<220>
   <221> misc_feature
   <222> (22)..(22)
   <223> n is a, c, g, or t
<220>
   <221> misc_feature
   <222> (25)..(25)
   <223> n is a, c, g, or t
<220>
   <221> misc_feature
   <222> (31)..(31)
   <223> n is a, c, g, or t
<400> 5
   tgtaaaacga cggccagtrc angangcraa narrc 35
<210> 6
   <211> 18
   <212> DNA
   <213> Actinomadura namibiensis
<400> 6
   agtgctgtag cacgggaa 18
<210> 7
   <211> 21
   <212> DNA
   <213> Artificial
<220>
   <223> Reversed Primer of Labyrinthopeptin A2
<220>
   <221> misc_feature
   <222> (7)..(7)
   <223> n is a, c, g, or t
<220>
   <221> misc_feature
   <222> (13)..(13)
   <223> n is a, c, g, or t
<400> 7
   rcarcangcr aanarrcttc c 21
<210> 8
   <211> 35
   <212> DNA
   <213> Artificial
<220>
   <223> Reversed Primer of Labyrinthopeptin A2
<400> 8
   cacggtacct agactagtga ccaagtgcgc cggtc 35
<210> 9
   <211> 22
   <212> DNA
   <213> Actinomadura namibiensis
<400> 9
   cttcccgtgc tacagcactc cc 22
<210> 10
   <211> 20
   <212> DNA
   <213> Artificial
<220>
   <223> Dig-labelled probe
<400> 10
   atggacctcg ccacgggctc 20
<210> 11
   <211> 22
   <212> DNA
   <213> Artificial
<220>
   <223> Dig-labelled probe
<400> 11
   cttcccgtgc tacagcactc cc 22
<210> 12
   <211> 159
   <212> DNA
   <213> Artificial
<220>
   <223> orf for Labyrinthopeptin A2
<400> 12
   tgacgcccgc acaccgttcc accgatgaga ggtgacagtc ccatggcgtc gatcctggaa 60
   ctccagaacc tggacgtcga gcacgcccgc ggcgagaacc gctccgactg gagcctgtgg 120
   gagtgctgta gcacgggaag cctgttcgcc tgctgctga 159
<210> 13
   <211> 117
   <212> DNA
   <213> Artificial
<220>
   <223> structural gene of prepro-Labyrinthopeptin A2 followed by stop-colon TGA
<400> 13
   atggcgtcga tcctggaact ccagaacctg gacgtcgagc acgcccgcgg cgagaaccgc 60
   tccgactgga gcctgtggga gtgctgtagc acgggaagcc tgttcgcctg ctgctga 117
<210> 14
   <211> 38
   <212> PRT
   <213> Artificial
<220>
   <223> Prepro-Labyrinthopeptin A2
<400> 14
   Met Ala Ser Ile Leu Glu Leu Gln Asn Leu Asp Val Glu His Ala Arg
   1 5 10 15
   Gly Glu Asn Arg Ser Asp Trp Ser Leu Trp Glu Cys Cys Ser Thr Gly
   20 25 30
   Ser Leu Phe Ala Cys Cys
   35
<210> 15
   <211> 18
   <212> PRT
   <213> Artificial
<220>
   <223> Pro-Labyrinthopeptin A2
<400> 15
   Ser Asp Trp Ser Leu Trp Glu Cys Cys Ser Thr Gly Ser Leu Phe Ala
   1 5 10 15
   Cys Cys
<210> 16
   <211> 135
   <212> DNA
   <213> Artificial
<220>
   <223> orf for Labyrinthopeptin A1
<400> 16
   tgaacatcca ccatggcatc catccttgag ctccaggacc tggaggtcga gcgcgccagc 60
   tcggccgccg acagcaacgc cagcgtctgg gagtgctgca gcacgggcag ctgggttccc 120
   ttcacctgct gctga 135
<210> 17
   <211> 123
   <212> DNA
   <213> Artificial
<220>
   <223> structural gene of prepro-Labyrinthopeptin A1 followed by stop-colon TGA
<400> 17
   atggcatcca tccttgagct ccaggacctg gaggtcgagc gcgccagctc ggccgccgac      60
   agcaacgcca gcgtctggga gtgctgcagc acgggcagct gggttccctt cacctgctgc     120
   tga                                                                   123
<210> 18
   <211> 40
   <212> PRT
   <213> Artificial
<220>
   <223> Prepro-Labyrinthopeptin A1
<400> 18
   Met Ala Ser Ile Leu Glu Leu Gln Asp Leu Glu Val Glu Arg Ala Ser
   1               5                   10                  15
   Ser Ala Ala Asp Ser Asn Ala Ser Val Trp Glu Cys Cys Ser Thr Gly
               20                  25                  30
   Ser Trp Val Pro Phe Thr Cys Cys
           35                  40
<210> 19
   <211> 20
   <212> PRT
   <213> Artificial
<220>
   <223> Pro-Labyrinthopeptin A1
<400> 19
   Ser Asn Ala Ser Val Trp Glu Cys Cys Ser Thr Gly Ser Trp Val Pro
   1               5                   10                  15
   Phe Thr Cys Cys
               20